(19) **Europäisches Patentamt / European Patent Office / Office européen des brevets**

(11) **EP 1 692 165 B1**

(12) **EUROPEAN PATENT SPECIFICATION**

(45) Date of publication and mention
of the grant of the patent:
**07.01.2009 Bulletin 2009/02**

(51) Int Cl.:
*C07K 14/47* (2006.01)     *G01N 33/50* (2006.01)
*C12N 15/10* (2006.01)     *C12Q 1/68* (2006.01)

(21) Application number: **04810260.2**

(22) Date of filing: **02.11.2004**

(86) International application number:
**PCT/US2004/036595**

(87) International publication number:
**WO 2005/042574 (12.05.2005 Gazette 2005/19)**

(54) **HIGH THROUGHPUT FUNCTIONAL ASSAY FOR G-PROTEIN COUPLED RECEPTORS USING A RAP-RAS CHIMERIC PROTEIN**

FUNKTIONELLER ASSAY MIT HOHEM DURCHSATZ FÜR G-PROTEIN-GEKOPPELTE REZEPTOREN MIT EINEM CHIMÄREN RAP-RAS-PROTEIN

DOSAGE FONCTIONNEL A HAUT RENDEMENT DES RECEPTEURS COUPLES A LA PROTEINE G UTILISANT UNE PROTEINE CHIMERIQUE RAP-RAS

(84) Designated Contracting States:
**AT BE BG CH CY CZ DE DK EE ES FI FR GB GR HU IE IS IT LI LU MC NL PL PT RO SE SI SK TR**

(30) Priority: **03.11.2003 US 517143 P**

(43) Date of publication of application:
**23.08.2006 Bulletin 2006/34**

(73) Proprietor: **Acadia Pharmaceuticals Inc.**
**San Diego, CA 92121 (US)**

(72) Inventors:
 • BURSTEIN, Ethan S.
 San Diego, CA 92130 (US)
 • PIU, Fabrice
 San Diego, CA 92131 (US)
 • MA, Jian-Nong
 San Diego, CA 92121 (US)
 • WEISSMAN, Jacques Thomas
 Carlsbad, CA 92008 (US)
 • WEINER, David, M.
 San Diego, CA 92191 (US)
 • SCULLY, Audra, L.
 Carlsbad, CA 92009 (US)
 • NASH, Norman
 San Diego, CA 92130 (US)
 • SPALDING, Tracy
 San Diego, CA 92130 (US)
 • CURRIER, Erika
 San Diego, CA 92117 (US)

(74) Representative: **Plougmann & Vingtoft A/S**
**Sundkrogsgade 9**
**P.O. Box 831**
**2100 Copenhagen Ø (DK)**

(56) References cited:
WO-A-00/40717     WO-A-02/20749
WO-A-97/48820     US-A- 5 912 132
US-A1- 2004 253 675     US-B1- 6 462 178

• MATSUBARA KENJI ET AL: "Plasma membrane recruitment of RalGDS is critical for Ras-dependent Ral activation" ONCOGENE, vol. 18, no. 6, 11 February 1999 (1999-02-11), pages 1303-1312, XP002316595 ISSN: 0950-9232
• MOLINARI P ET AL: "Promiscuous coupling at receptor-Galpha fusion proteins. The receptor of one covalent complex interacts with the alpha-subunit of another" JOURNAL OF BIOLOGICAL CHEMISTRY, AMERICAN SOCIETY OF BIOLOGICAL CHEMISTS, BALTIMORE, MD, US, vol. 278, no. 18, 2 May 2003 (2003-05-02), pages 15778-15788, XP002297484 ISSN: 0021-9258
• HATTORI MASAKAZU ET AL: "Rap1 GTPase: Functions, regulation, and malignancy." JOURNAL OF BIOCHEMISTRY (TOKYO), vol. 134, no. 4, October 2003 (2003-10), pages 479-484, XP002316685 ISSN: 0021-924X

**(Cont. next page)**

• **WEISSMAN JACQUES T ET AL: "G-protein-coupled receptor-mediated activation of rap GTPases: Characterization of a novel Galphai regulated pathway." ONCOGENE, vol. 23, no. 1, 8 January 2004 (2004-01-08), pages 241-249, XP002316594 ISSN: 0950-9232**

## Description

Field of the Invention

The invention relates to a G-protein coupled receptor high-throughput functional assay for screening candidate molecules for their ability to activate or inhibit a G-protein coupled receptor.

Background of the Invention

The G-protein coupled receptor (GPCR) family is the largest known gene family representing greater than 1% of the human genome (1). G-protein coupled receptors are also the most exploited gene family by the pharmaceutical industry for the development of therapeutic compounds. The molecular diversity and discrete tissue localizations of many GPCRs provides opportunities to develop selective, efficacious compounds with favorable side-effect profiles. As approximately 50% of the GPCRs are considered to be 'orphan' receptors, there clearly exists a large untapped drug discovery potential.

A commonly employed strategy to identify novel lead compounds is high-throughput screening of diverse chemical libraries. Assays used are either based upon measuring compound binding to defined molecular targets or measuring functional outputs resulting from compound/receptor interactions. A major limitation of competition binding assays is that they cannot be used to screen orphan receptors. In contrast to binding assays, functional assays are amenable to screening orphan receptors assuming that the functional outputs can be detected. In addition, functional assays allow the determination of intrinsic activities of ligands (2). Thus there is strong incentive to develop high-throughput functional assays with broad applicability across the GPCR family.

Developing functional assays with broad applicability is problematical due to the heterogeneity of responses elicited by GPCRs. Most functional assays for GPCRs involve measuring second messenger levels either directly or through the use of reporter constructs. Examples of second messengers commonly measured to assess GPCR function include inositol phosphates, calcium, and cyclic AMP (3-8). Assay protocols to measure these second messengers typically require expensive reagents, radiolabeled compounds, and/or numerous laborious steps to quantify responses. Another disadvantage is that due to the heterogeneity of these measured responses, most functional assays are limited in scope to discreet subsets of GPCRs and require *a priori* knowledge of the signal-transduction properties to provide reliable screens for orphan receptors.

GPCRs can be crudely divided in receptors that stimulate cyclic AMP production through PTX-insensitive G-proteins (Gs-coupled), receptors that inhibit cyclic AMP production through PTX-sensitive G-proteins (Gi-coupled) and receptors that stimulate phosphatidyl inositol production through PTX-insensitive G-proteins (Gq-coupled). For example, assays that measure calcium flux work well with receptors coupled to Gq-family G-proteins but marginally well with receptors coupled to Gi-family G-proteins and not at all with receptors coupled to Gs-family G-proteins. Likewise, cyclic AMP measurements work for Gs and Gi-coupled receptors, but not Gq-coupled receptors.

Functional assays for Gs-coupled receptors either quantify cAMP production directly (6), the transcriptional regulation of reporter genes by cyclic AMP response (CRE) element promoter (7), or pigment change in frog melanophores (8). Unlike measuring calcium flux, cyclic AMP assays lack the throughput needed to perform HTS effectively, thus there is a need for a high-throughput functional assay that is compatible with Gs-coupled receptors.

To convert the heterogeneous functional responses of GPCRs into homogeneous outputs researchers have used chimeric G-alpha subunits (9-12) that link Gi-coupled receptors to Gq-regulated responses and promiscuous (with respect to receptor coupling) G-alpha subunits (13,14) to link GPCRs to calcium flux. However possibly because Gs and the G12/13 G-proteins are more distantly related to Gi than Gq (15,16), chimeric G-proteins between these classes of Galpha subunits do not work as well as Gq/Gi chimeras (10). In addition, even Gi-linked receptors display distinct preferences for different Gq/Gi family chimeric constructs (11,12,17) thus the reliability of this strategy may vary significantly between receptors. Similarly, the so-called universally compatible Gα15 and Gα16 subunits do not couple universally well enough to all GPCRs (18,19) to be useful as a general enablement strategy. Thus, there still exists a need for high-throughput functional assays for GPCRs that are more universally compatible and in particular amenable to Gs-linked receptors.

An alternate strategy to link GPCR signaling to a common, measurable output is to employ genes that lie downstream from the heterotrimeric G-proteins. Besides activating heterotrimeric G-proteins, GPCRs also activate ras-related GTPases which in turn mediate a diverse array of cellular effects on growth, differentiation, morphology, and gene expression (20). With respect to the heterotrimeric G-proteins, the ras-related GTPases lie downstream in the signal transduction cascade. In all cases known, the final step linking activation of GPCRs to activation of ras-related GTPases is activation of a guanine nucleotide exchange factor (GEF). GEFs catalyze exchange of GTP for GDP leading to GTPase activation and exhibit substrate specificity for distinct sub-families of ras-related GTPases (21,22).

Gq, Gs, and Gi linked GPCRs are known to activate the ras-related GTPase rap (23-27), although through distinct signal transduction pathways. Furthermore, the second messengers calcium, diacylglycerol, and cyclic AMP, are known to directly activate GEFs selective for rap GTPases (27-29). Therefore, rap GTPases were used to link GPCRs to a common, measurable output.

Summary of the Invention

One aspect of the present invention is a method for enabling or improving assays of gene function using co-expression of helper genes. In one embodiment, the method is for the purposes of identifying chemical compounds which bind to gene products, and modulate their function positively or negatively. In another embodiment, the method is for the purposes of detecting/validating the function of genes whose functions or abilities to function are unknown. In still another embodiment, the method is for the purposes of identifying the signal transduction properties of genes whose functions or abilities to function are unknown and thereby optimizing drug discovery screening assays for those genes.

In some embodiments, the genes being assayed are receptors. In some embodiments, the genes being assayed are G-protein coupled receptors. In some embodiments, the assays of gene function measure changes in gene expression. In some embodiments, the assays of gene function measure changes in second messenger levels. In some embodiments, the assays of gene function measure changes in cellular growth, morphology, differentiation, or survival.

In some embodiments, the helper genes enable a response to receptor activation that the receptor does not normally produce. In some embodiments, the helper genes amplify responses that the receptor does normally produce. In some embodiments, the helper genes amplify responses that the receptor does not normally produce but that are enabled by other helper genes. In some embodiments, the helper genes block receptor responses that interfere with detection of the primary functional response. In some embodiments, the helper genes contain mutations which block interfering signal inputs or outputs while preserving or enhancing the primary function response. In some embodiments, the helper genes are chimeras between 2 or more genes that redirect signal transduction pathways, linking domains that receive regulatory or signal inputs to domains that provide effector or signal outputs. In some embodiments, the chimeric helpers are comprised of domains derived from different G-proteins. In some embodiments, the chimeric helpers are comprised of domains derived from different G-proteins of the ras-superfamily. In some embodiments, the chimeric helpers are comprised of domains derived from different G-proteins of the rap subfamily and the ras subfamily. In some embodiments, the helper genes are naturally occurring genes that are not normally expressed in the host cell used for the functional assay. In some embodiments, the helper genes are naturally occurring genes that are normally expressed in the host cell used for the functional assay that are overexpressed. In some embodiments, the helper genes are truncated versions of naturally occurring genes that are not normally expressed in the host cell used for the functional assay. In some embodiments, the helper genes are truncated versions of naturally occurring genes that are normally expressed in the host cell used for the functional assay. In some embodiments, the helper genes are chimeras that additionally contain mutations not naturally occurring within either gene from which the chimeras that comprise the chimera are derived. In some embodiments, the helper genes are naturally occurring genes that are not normally expressed in the host cell used for the functional assay that additionally contain mutations not naturally occurring within those genes. In some embodiments, the helper genes are naturally occurring genes that are normally expressed in the host cell used for the functional assay that additionally contain mutations not naturally occurring within those genes. In some embodiments, the helper genes are mixtures of 2 or more genes, chimeras, mutant genes, or truncated genes which when co-expressed enable or improve detection of functional responses to receptors. In some embodiments, the helper genes are other naturally occurring receptors that help the receptor being functionally assayed to signal better. In some embodiments, the helper genes are other naturally occurring receptors that help the expression and formation of the receptor being functionally assayed. In some embodiments, the helper genes are other naturally occurring receptors that help the receptor being functionally assayed to respond more sensitively to ligands. In some embodiments, the helper genes are other unnaturally occurring receptors or mutant receptors, or fragments of receptors, that help the receptor being functionally assayed to signal better. In some embodiments, the helper genes are other unnaturally occurring receptors or mutant receptors, or fragments of receptors, that help the expression and formation of the receptor being functionally assayed. In some embodiments, the helper genes are other unnaturally occurring receptors or mutant receptors, or fragments of receptors, that help the receptor being functionally assayed to respond more sensitively to ligands.

Brief Description of the Drawings

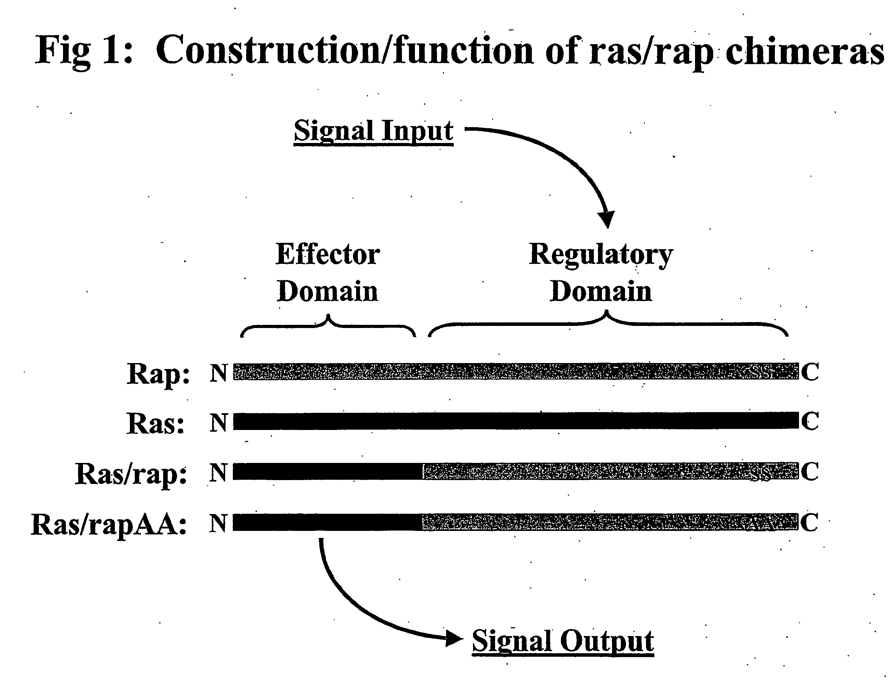

**Figure 1.** Construction of ras/rap chimeras which link rap inputs to ras outputs.

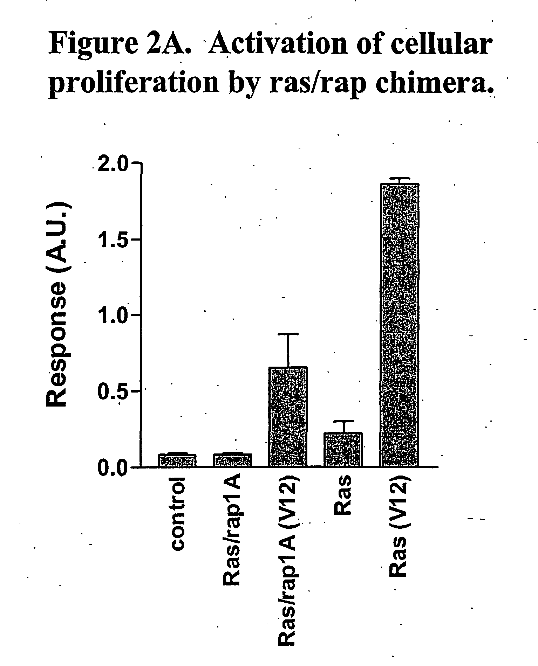

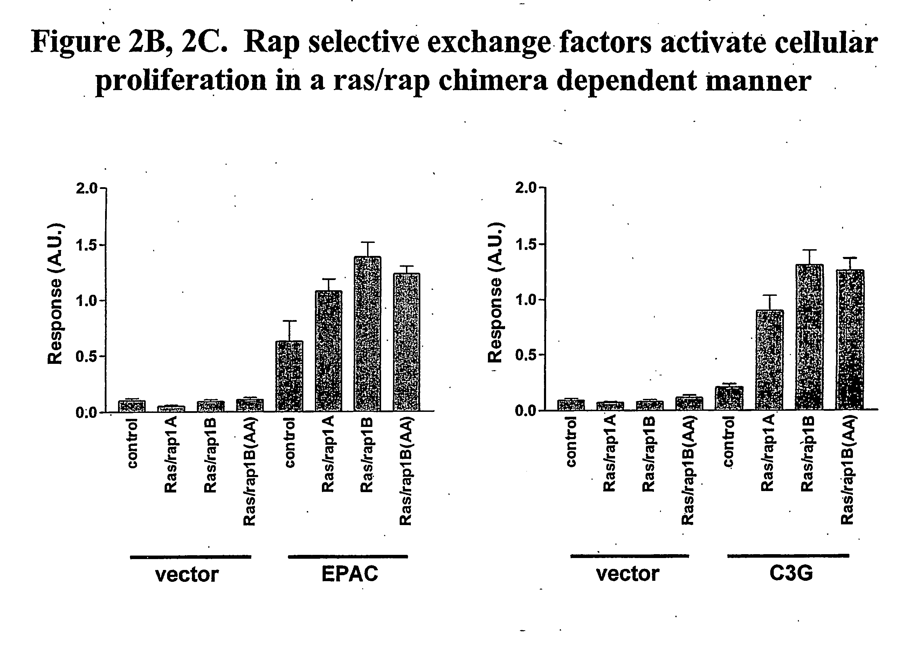

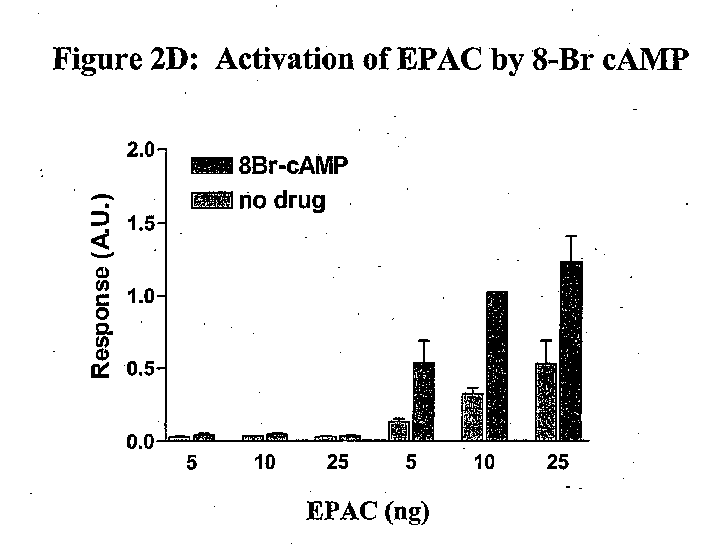

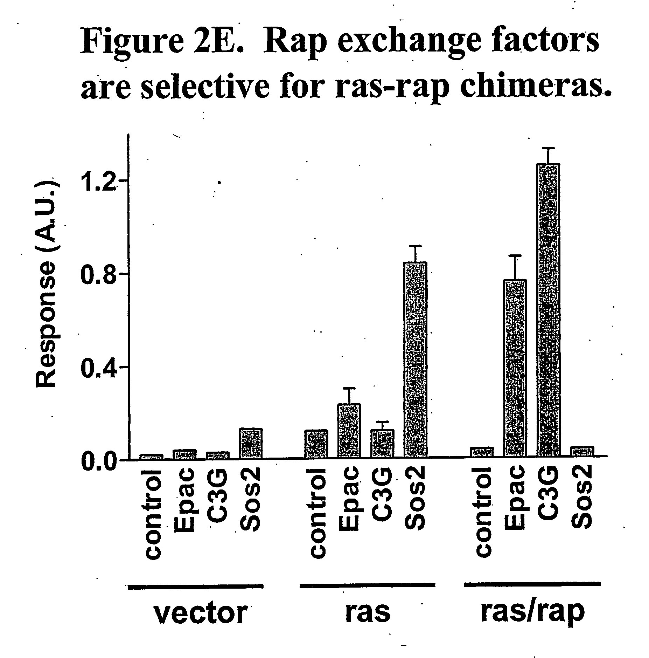

**Figure 2.** Characterization of regulation and signaling properties of ras/rap chimeras. **A**) Proliferative responses of ras, rap, and ras/rap measured in R-SAT. 4 ng/well each of wild-type or GTPase deficient (V12) mutants of ras, rap1A, or ras/rap1A (24) were transfected and analyzed for proliferative responses as described in Methods. **B & C**) Rap-selective exchange factors activate cellular proliferation in a ras/rap chimera dependent manner. **B**) Synergy of EPAC with ras/rap chimeras. Two ng per well of the rap selective exchange factor EPAC was co-expressed with 20 ng per well of either rap1B, ras/rap1A, ras/rap1B, ras/rapAA, or control vector and analyzed for proliferative responses as described above. **C**) Synergy of C3G with ras/rap chimeras. Twenty ng per well of the rap selective exchange factor C3G (68) was co-expressed with 20 ng per well of either rap1B ras/rap1A, ras/rap1B, ras/rapAA, or control vector and analyzed for proliferative responses as described above. **D**) The indicated doses (ng per well) of EPAC were co-expressed with 20 ng per well of ras/rapAA in the presence or absence of 250uM 8-Br cAMP and analyzed for proliferative responses as described above. **E**) Rap exchange factors are selective for ras/rap chimeras over ras. Either 10 ng per well of control vector, ras or ras/rapAA was co-expressed with EPAC (2 ng per well), C3G (10 ng per well), or Sos2 (10 ng per well) and analyzed for proliferative responses.

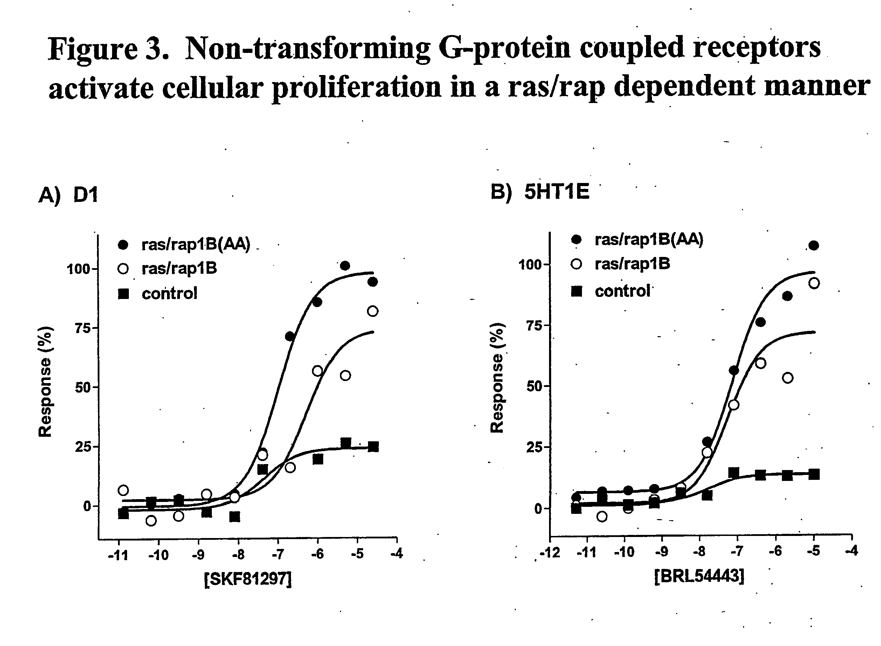

**Figure 3**. Gs-coupled and Gi-coupled GPCRs activated cellular proliferation in a ras/rap dependent manner. **A**) The D1 dopamine receptor (4 ng per 96 well) was co-expressed with 20ng per well of control vector, ras/rap1B, or ras/rapAA and analyzed for proliferative responses in the presence of the indicated concentrations of SKF81297 as described above. **B**) The 5HT1E serotonin receptor (20 ng per well) was co-expressed with 20 ng per well of control vector, ras/rap1B, or ras/rapAA and analyzed for proliferative responses in the presence of the indicated concentrations of BRL54443.

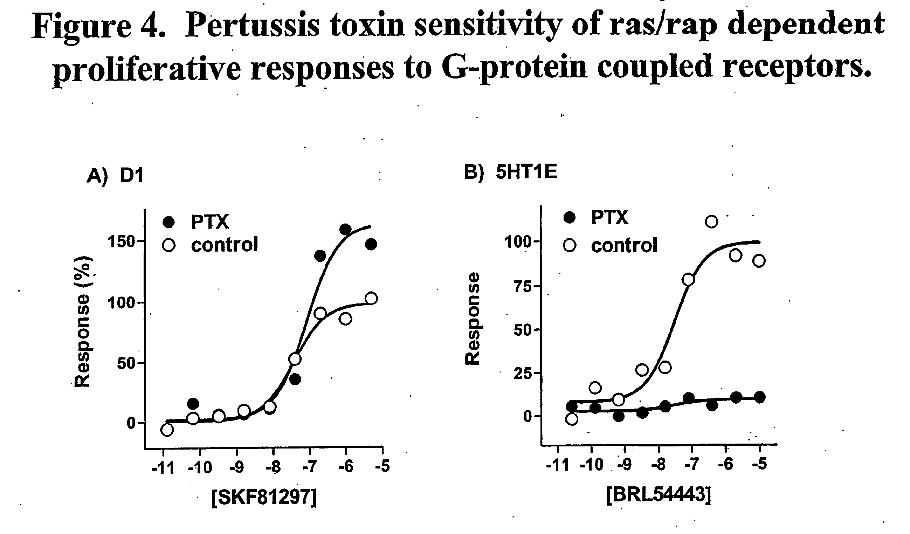

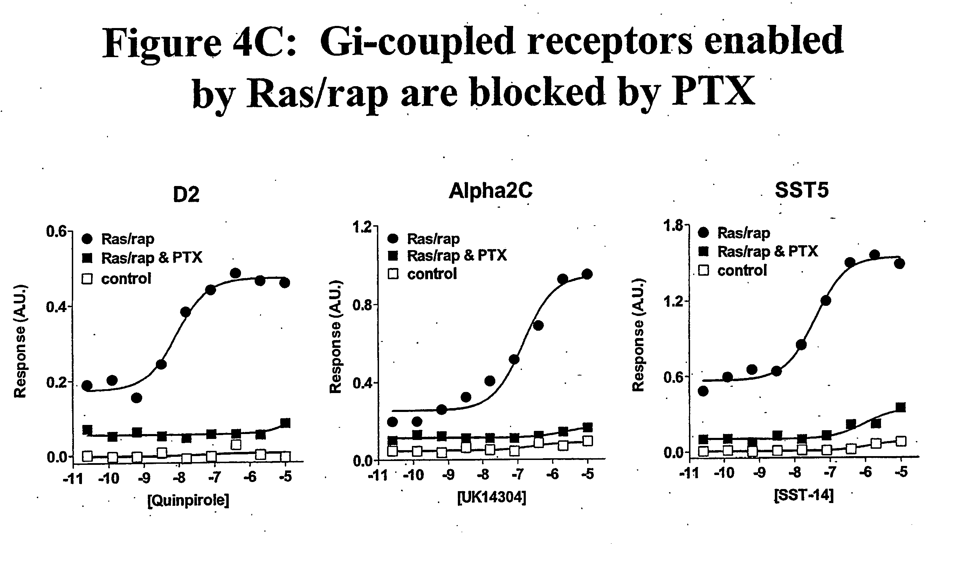

**Figure 4**. Pertussis toxin sensitivity of ras/rap dependent proliferative responses to Gs-coupled and Gi-coupled GPCRs. **A**) D1 (4 ng per well) and ras/rapAA (20 ng per well) were co-expressed and analyzed for proliferative responses in the presence or absence of 100 ug/ml pertussis toxin in the presence of the indicated concentrations of SKF81297. **B**) 5HT1E (20 ng per well) and ras/rapAA (20 ng per well) were co-expressed and analyzed for proliferative responses in the presence or absence of 100 ug/ml pertussis toxin in the presence of the indicated concentrations of BRL54443. C) D2 (4 ng/well), alpha2C (20 ng/well), and SST5 (4 ng/well) were each expressed with ras/rapAA (20 ng/well), incubated with the indicated concentrations of ligand and either with or without 100 ng/ml pertussis toxin (PTX) and analyzed for proliferative responses as described above. Control represents the receptors expressed alone.

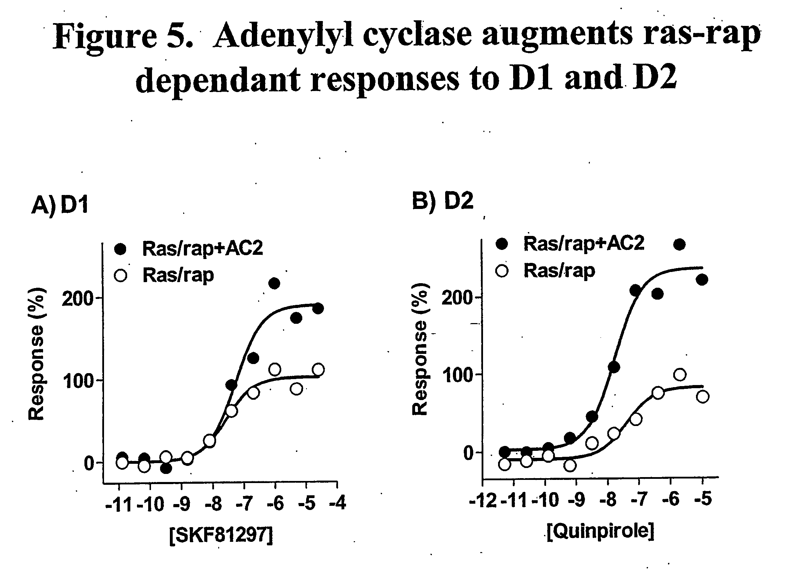

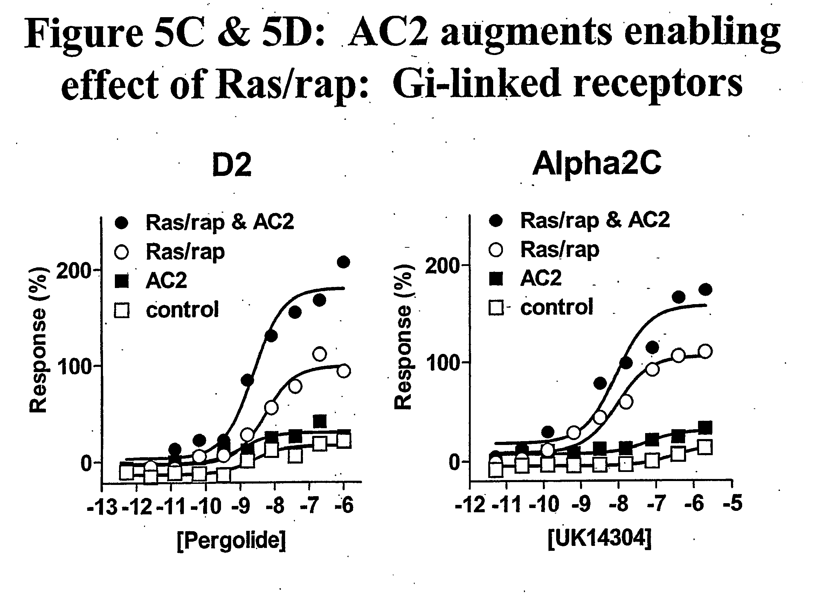

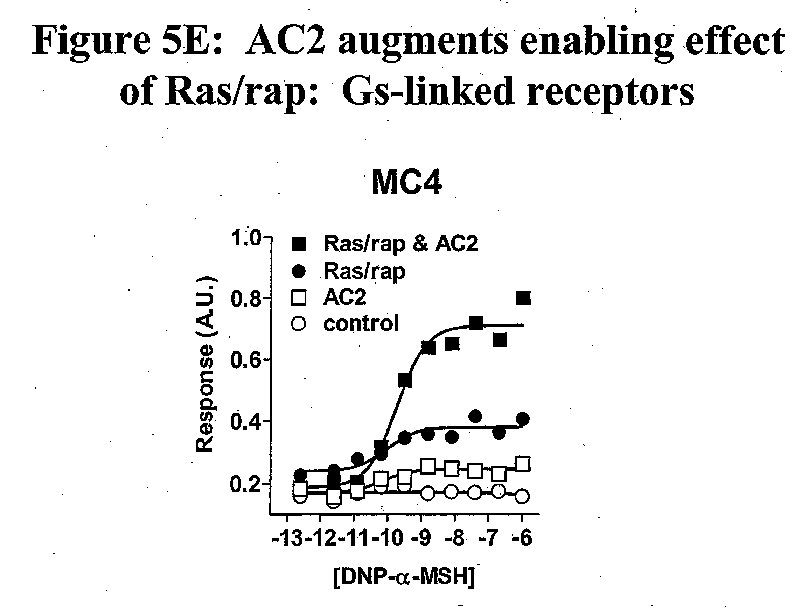

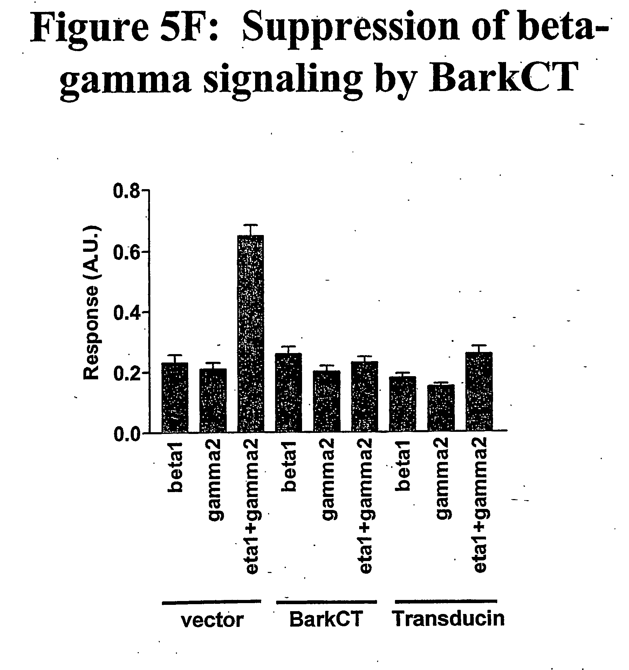

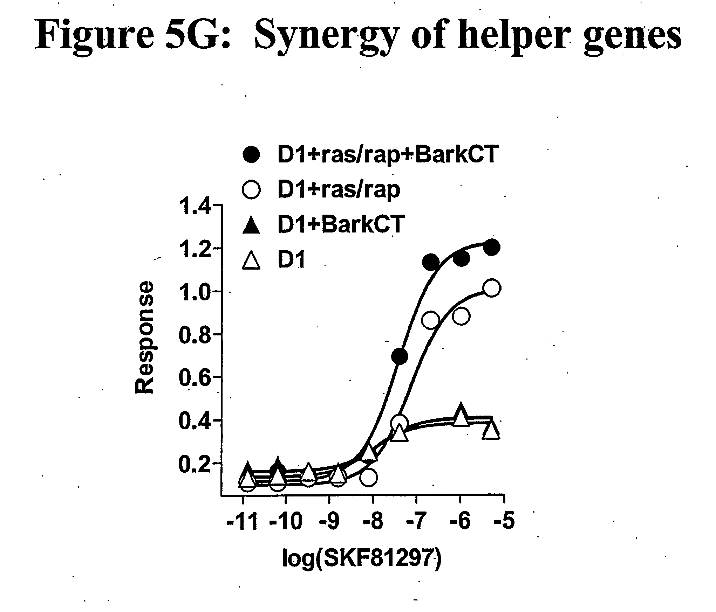

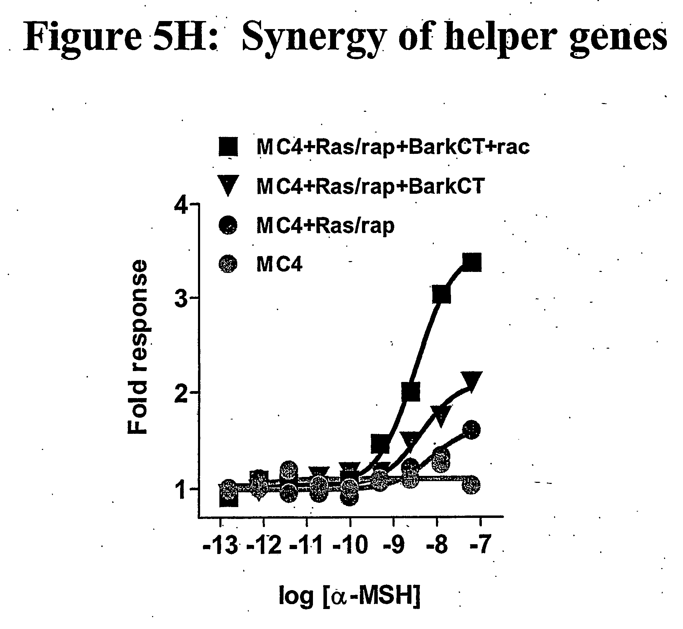

**Figure 5**. Other genes can be co-transfected to amplify the enabling effects of ras/rap. **A & B**) D1 and D2 (4 ng/well each) were transfected with ras/rapAA (20 ng/well) and with or without adenylyl cyclase type II (2 ng/well), incubated with the indicated concentrations of ligand and analyzed for proliferative responses as described above. **C & D**) D2 (4 ng/well) and alpha2C (20 ng/well) were each expressed alone, with adenylyl cyclase type II (2 ng/well), ras/rapAA (20 ng/well), or both and analyzed for proliferative responses as described above. **E**) MC4 (20 ng/well) was expressed alone, with adenylyl cyclase type II (2 ng/well), ras/rapAA (20 ng/well), or both and analyzed for proliferative responses as described above. **F**) The beta1 and gamma2 (2 ng each per well) subunits were expressed alone or together with either control vector, BarkCT, or the alpha subunit of transducin. Control vector was added to balance the pool size in each transfection. **G**) D1 (4 ng/well) was expressed alone, with BarkCT (4 ng/well), ras/rapAA (20 ng/well), or both and analyzed for proliferative responses as described above. **H**) MC4 (20 ng/well) was expressed alone, with ras/rapAA (20 ng/well), with BarkCT (4 ng/well) and ras/rapAA (20 ng/well), or with BarkCT (4 ng/well), ras/rapAA (20 ng/well), and rac (4 ng/well) and analyzed for proliferative responses as described above.

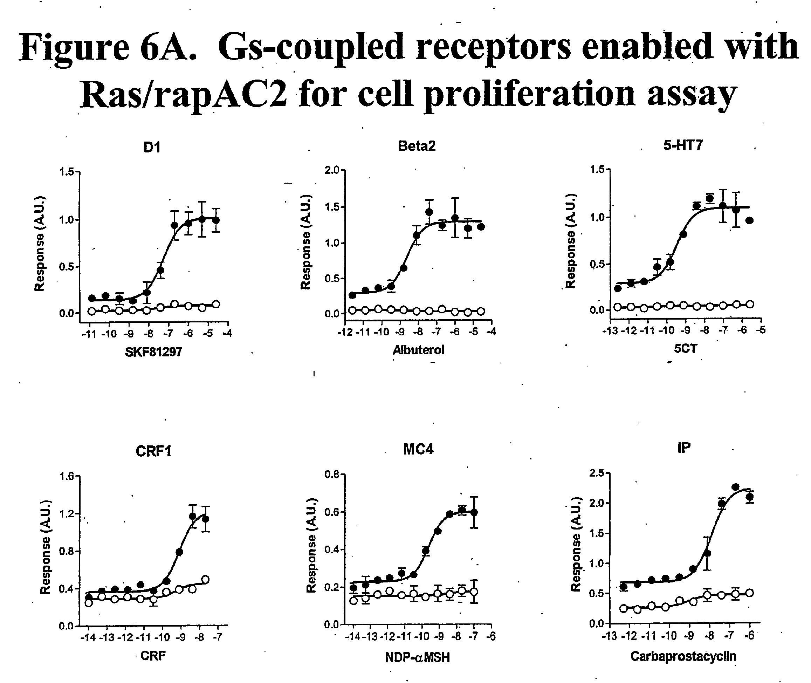

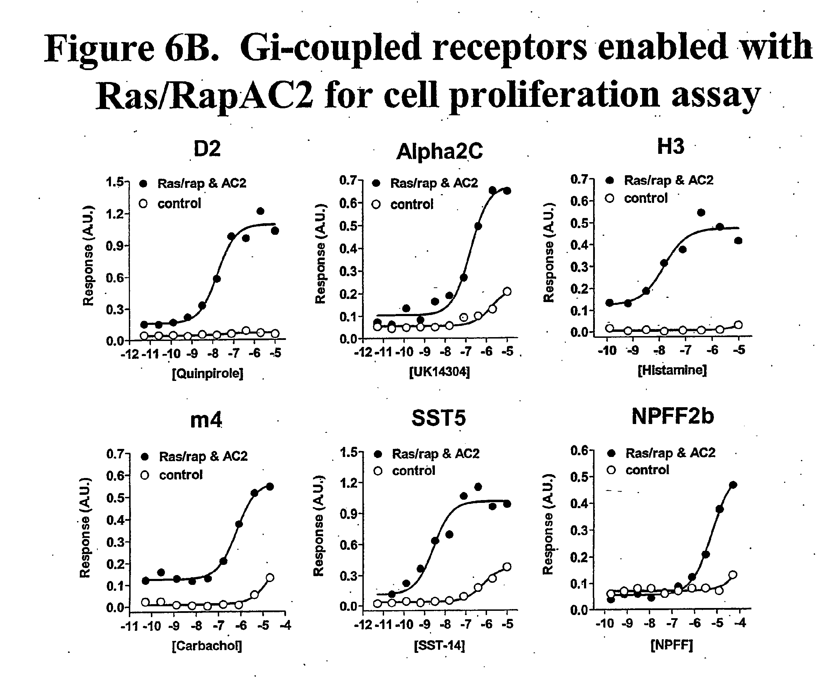

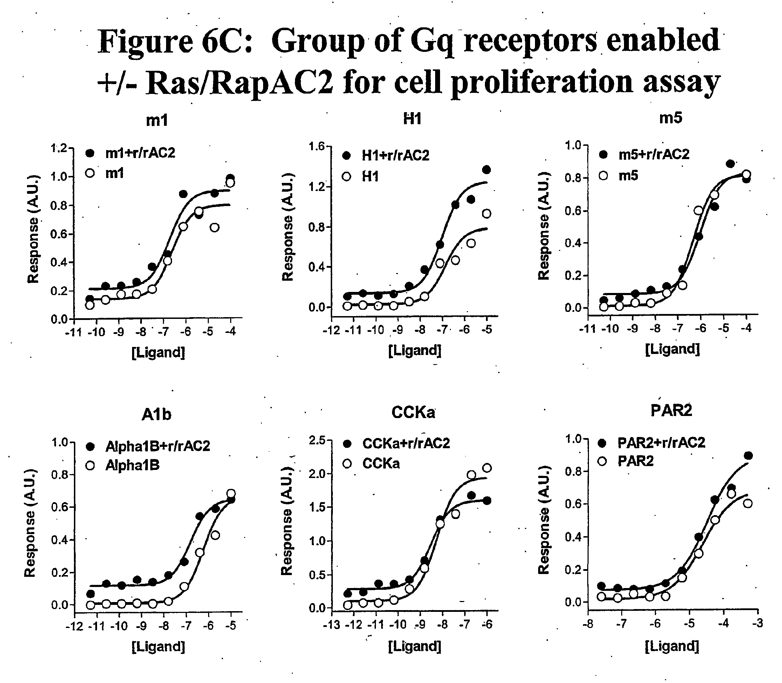

**Figure 6**. GPCRs stimulate proliferative responses in the presence of helper genes. **A**) The indicated Gs-coupled receptors and beta-galactosidase were transiently transfected into NIH3T3 cells either with or without ras/rap (20 ng/well) and AC2 (2 ng/well) and assayed for proliferative responses in the presence of the indicated concentrations of ligand as described above in the Materials and Methods. **B**) The indicated Gi-coupled receptors and beta-galactosidase were transiently transfected into NIH3T3 cells either with or without ras/rap (20 ng/well) and AC2 (2 ng/well) and assayed for proliferative responses in the presence of the indicated concentrations of ligand as described above in the Materials and Methods. **C**) The indicated Gq- G12/13-coupled receptors and beta-galactosidase were transiently transfected into NIH3T3 cells either with or without ras/rap (20 ng/well) and AC2 (2 ng/well) and assayed for proliferative responses in the presence of the indicated concentrations of ligand as described above in the Methods.

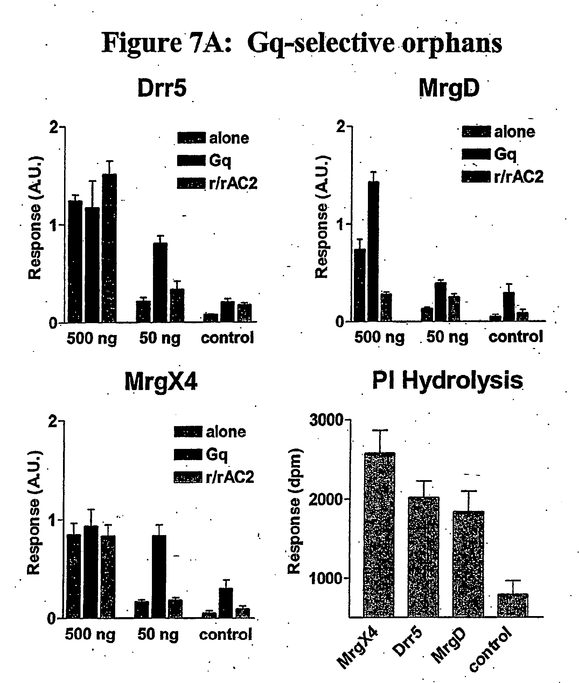

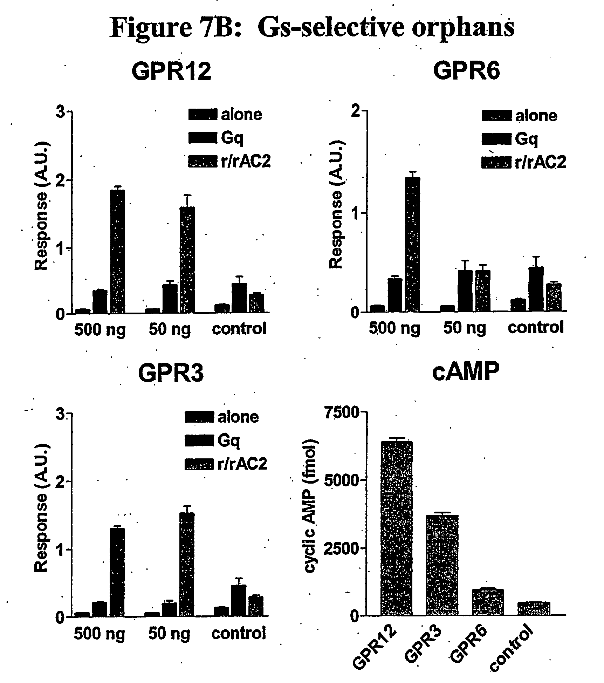

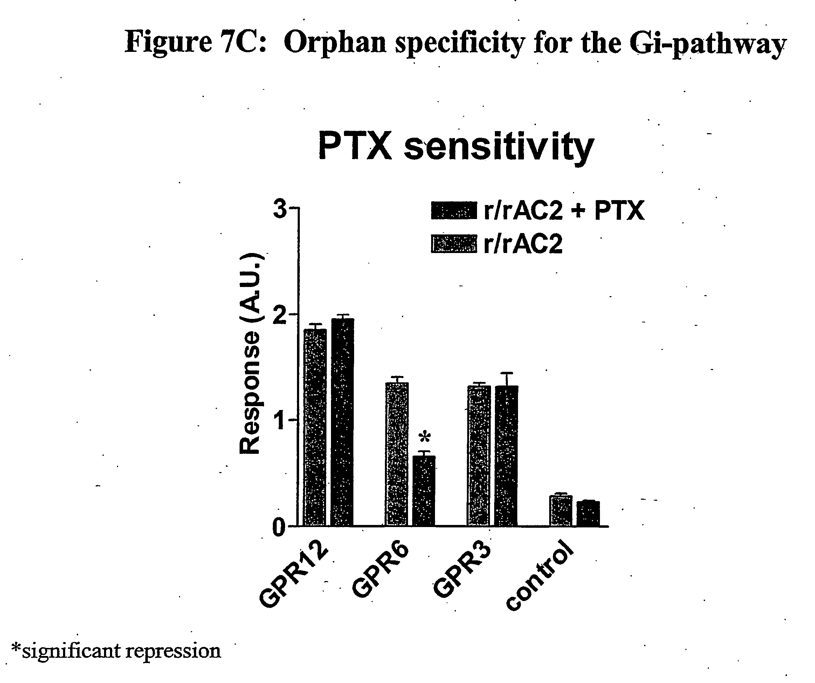

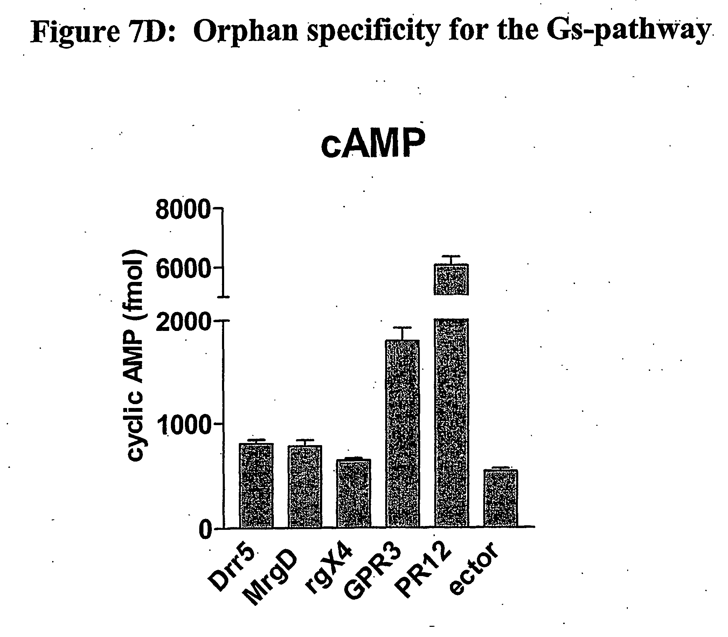

**Figure 7.** Functional validation and characterization of orphan GPCRs using helper genes. A) The indicated receptors were expressed at two doses either alone, with ras/rapAA (20ng/well) and ACII (2ng/well), or G$\alpha$q (4 ng/well) and analyzed for constitutive stimulation of cellular proliferation as described above. Control represents the transfection of the same helper genes with vector substituting for receptor. The transfections were all balanced with vector to ensure the same total amount of DNA was transfected in each case. PI hydrolysis assays were carried out using tsa cells transfected with 20 ng/well of each receptor as described in Methods. **B**) The indicated receptors were expressed at two doses either alone, with ras/rapAA (20ng/well) and ACII (2ng/well), or G$\alpha$q (4 ng/well) and analyzed for constitutive stimulation of cellular proliferation as described above. Control represents the transfection of the same helper genes with vector substituting for receptor. The transfections were all balanced with vector to ensure the same total amount of DNA was transfected in each case. Cyclic AMP accumulation assays were carried out using HEK293 cells transfected with 20 ng/well of each receptor as described in Methods. **C**) The indicated receptors were expressed at with ras/rapAA (20ng/well) and ACII (2ng/well), incubated in the presence or absence of pertussis toxin (PTX, 100 ng/ml) and analyzed for constitutive stimulation of cellular proliferation as described above. Control represents the transfection of the same helper genes with vector substituting for receptor. **D**) The indicated receptors were tested for stimulation of cyclic AMP production as described in the experimental methods.

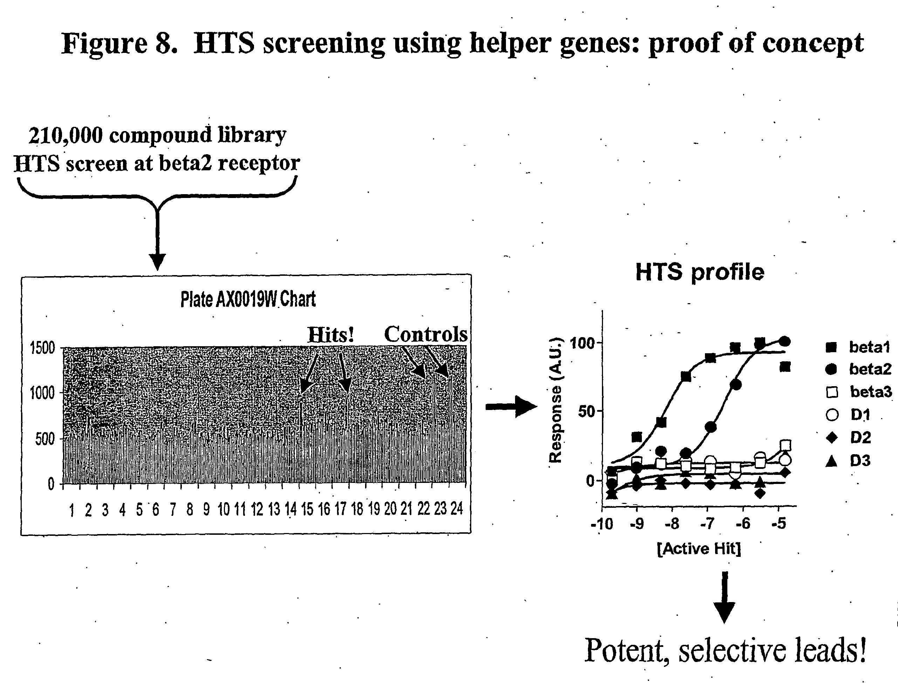

**Figure 8.** Results of a HTS screen. NIH3T3 cells were transiently transfected with the beta2 receptor, 5HT7 receptor, and the secretin receptor and a mixture of ras/rapAA, ACII, BarkCT, rac, and beta-galactosidase and used to screen a library of 210,000 compounds for agonist activity at these receptors. Hits and positive controls are indicated. Hits were retested against each individual receptor. Hits displaying selectivity were selected for further pharmacological characterization as shown.

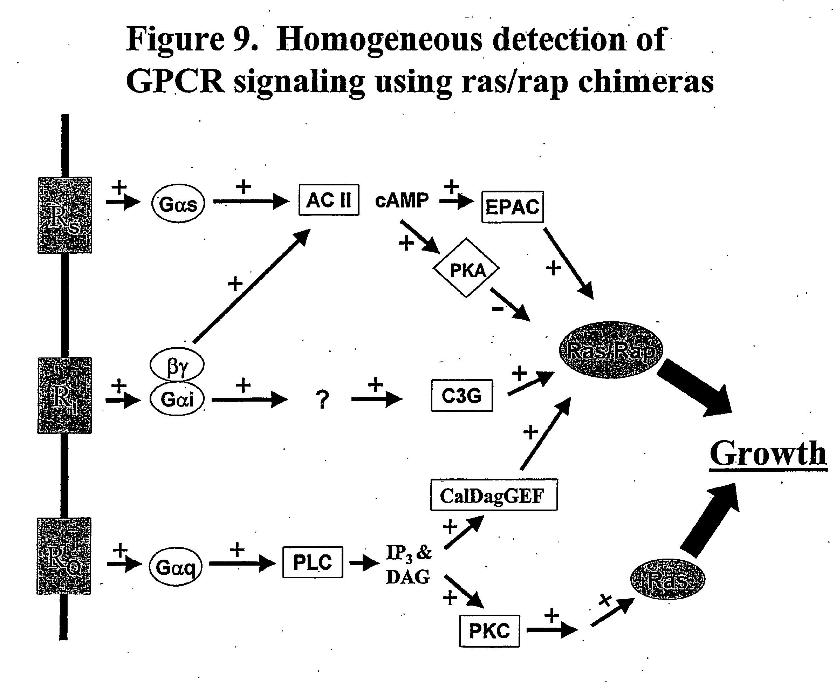

**Figure 9.** Shows a schematic of GPCR signaling pathways using ras/rap chimeras.

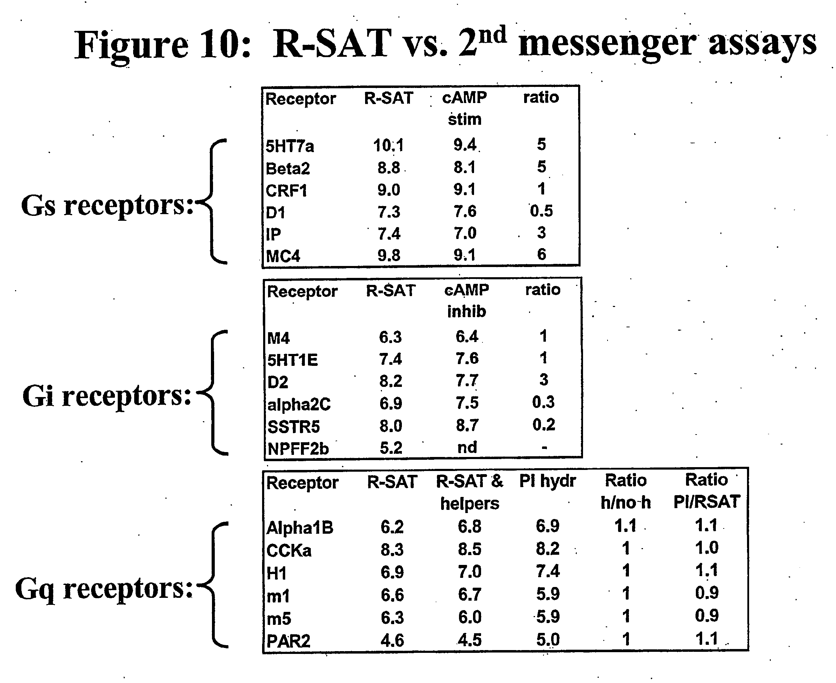

**Figure 10.** Comparison of cellular proliferation assay (R-SAT™, see U.S. Patent No. 5,707,798, which is hereby incorporated herein by reference in its entirety) with second messenger assays for cyclic AMP production and phosphotidyl inositol (PI) hydrolysis. The indicated receptors were tested for functional responses in the cellular proliferation assay or the indicated second messenger assay. Shown are the $pEC_{50}$ values obtained in each assay and the ratio of these values.

Detailed Description of the Preferred Embodiment

The G-protein coupled receptors (GPCR) superfamily is the most exploited gene family for drug discovery. Given that, and that over 50% of the GPCR family are classified as 'orphan receptors', there is a need to develop high-throughput functional assays that have broad applicability across this family. Due to the heterogenous nature of functional responses to GPCRs, most assays of GPCR function only work for discreet subsets of GPCRs. We now report that robust responses to Gs- and Gi-coupled GPCRs can readily be measured through use of chimeras linking the regulatory domain of the rap1B protein to the effector region of the ras oncogene, in conjuction with existing functional assays for cellular proliferation. We further show that overexpression of other genes can further augment the enabling properties of ras/rap chimeras. Collectively this cocktail of enabling and amplifying genes are termed 'helper genes'. In addition, the presence of 'helper genes' does not significantly alter the functional responses to Gq and G12/13 coupled GPCRs in these same assay formats. Thus the use of this helper gene enablement strategy in conjunction with existing functional assays provides a nearly universal assay of GPCR function. The utility of this invention for drug screening and for the enablement and prediction of functional properties of orphan receptors is demonstrated.

The invention disclosed below relates to a functional assay for screening candidate compounds for activity against particular G-protein coupled receptors. In a preferred embodiment, a target cell is prepared that expresses a particular G-protein coupled receptor (GPCR) of interest. The GPCR is functionally linked to a measurable output using rap/ras chimeric construct or other 'helper genes'.

One convenient measurable output is cellular proliferation. To demonstrate the utility of this approach, we employed a high-throughput cell-based functional assay (R-SAT™, Receptor Selection and Amplification Technology) as a convenient means to test this enabling technology. Due to the simplicity of the assay, R-SAT™ is well suited to high-throughput applications (30). The abilities of genes to induce cellular proliferation in R-SAT are strongly correlated with their abilities to transform NIH-3T3 cells in focus forming assays. Therefore GPCRs coupled to Gαq and Gα12/13, as well as many protooncogenes such as ras induce strong responses in R-SAT™ whereas GPCRs coupled to the Gαi and Gas families of heterotrimeric G-proteins which do not transform NIH-3T3 cells also do not induce responses in R-SAT™ (10, 31, 32 and Burstein unpublished observations). By employing chimeras between rap GTPases and ras GTPases, which contain the effector functions of ras, and the regulatory domain of rap, we have discovered that signals derived from Gi and Gs-coupled receptors can be redirected to stimulate cellular proliferation, similar to Gq and G12/13 linked GPCRs, and that the pharmacological responses determined in this manner are highly predictive of results obtained with second messenger assays. We also demonstrate that these redirected signals can be further amplified by the overexpression of other 'helper' genes. Furthermore, we show that functional responses to GPCRs coupled to Gq or G12/13 pathways are not significantly altered by co-expression of ras/rap or the helper genes, thus most GPCRs can be assayed using a single assay format. Finally the utility of using ras/rap for functional characterization of orphan GPCRs is shown.

To link GPCRs to a common, measurable output we sought to employ the rap GTPases because Gq, Gs and Gi linked GPCRs are all known to activate rap (23-29). One convenient measurable output is cellular proliferation. Using chimeras between rap GTPases and ras GTPases, which contain the effector functions of ras, and the regulatory domain of rap, we have discovered that signals derived from Gi and Gs-coupled receptors can be redirected to stimulate cellular proliferation, similar to Gq and G12/13 linked GPCRs, and that their pharmacological responses determined in this manner are highly predictive of results obtained with second messenger assays. Furthermore, functional responses to GPCRs coupled to Gq or G12/13 pathways are not significantly altered by co-expression of the helper genes described above. As configured with helper genes, the assay retains all of the attributes that allow high-throughput functional analysis.

By measuring constitutive responses, the functionality and signaling properties of a panel of orphan GPCRs were determined, demonstrating the utility of the helper gene strategy for enabling and characterizing functional responses to orphan GPCRs. As described above, most GPCRs can be assayed using a single assay format, allowing for functional analysis of Gas, Gαi, and Gaq-coupled receptors, and unbiased screening of orphan GPCRs. A diagram summarizing homogeneous detection of GPCR signaling using ras/rap chimeras is shown in **Figure 9.**

To demonstrate the utility of our approach, we employed a high-throughput cell-based functional assay (R-SAT™, Receptor Selection and Amplification Technology), described in U.S. Patent No. 6,358,698, entitled "Methods of identifying inverse agonists of the serotonin 2A receptor;" U.S. Patent No. 5,955,281, entitled "Identification of ligands by selective amplification of cells transfected with receptors;" and U.S. Patent No. 5,912,132, entitled "Identification of ligands by selective amplification of cells transfected with receptors, all of which are hereby incorporated by reference in their entirety.

Due to the simplicity of the assay, R-SAT™ is well suited to high-throughput applications (30). R-SAT™ is based on the observation that oncogenes and many receptors induce proliferation or transformation responses in NIH-3T3 cells. As such, the abilities of genes to transform NIH-3T3 cells and to induce responses in R-SAT™ are strongly correlated. GPCRs coupled to Gαq or G12/13 (which induce focus formation in NIH-3T3 cells, see 46,47) as well as many growth factor receptors and non-receptor protooncogenes such as G-proteins and MAP kinases induce strong responses in R-SAT™ (10,32), whereas GPCRs coupled to the Gαi and Gαs families of heterotrimeric G-proteins do not transform NIH-3T3 cells and do not by themselves induce responses in R-SAT™. Cyclic AMP is known to mediate growth inhibitory signals in many cell types (48,49), including NIH3T3 cells (50), and therefore it is not surprising that GPCRs that promote production of cyclic AMP induce little or no response in R-SAT. These limitations have been overcome using the strategies outlined above. Thus, this work demonstrates that the R-SAT™ technology is not limited to genes that stimulate cellular proliferation.

Genes that operate downstream of heterotrimeric G-proteins have been utilized to enable homogeneous detection of robust pharmacological responses to GPCRs. Although it has been shown that signaling outputs in R-SAT™ and other functional assays can also be redirected to common final pathways through the use of chimeric heterotrimeric Galpha subunits (9-12) or through the use of the promiscuous Galpha subunits (Gα15 and Gα16) (14), these approaches suffer from several disadvantages. G-protein stoichiometry and hence receptor pharmacology is altered, receptor coupling to an artificial construct is measured, and both chimeric and promiscuous G-alpha subunits do not couple universally well enough to all GPCRs to be useful as a general enablement strategy (11,12,17-19). By employing genes that operate downstream of Galpha subunits to link signaling outputs from GPCRs to a common, measurable output, GPCR coupling to endogenous heterotrimeric Galpha subunits is measured, significantly improving the reliability and generality of the assays, and the fidelity of the pharmacology detected. Indeed, functional coupling of a wide variety of GPCRs including family A, family B, and family C receptors, and receptors which bind monoaminergic, lipid, and peptide ligands has been shown, and when compared to second messenger assays, a strong correlation of potencies is observed.

Among many gene families downstream of heterotrimeric G-proteins that link GPCRs to intracellular signal transduction, we exploited the low molecular weight, ras-related GTPases and the adenylyl cyclase gene family. The ras-related GTPases are known to play important roles in mediating the effects of GPCRs on cellular growth and metabolism, linking heterotrimeric G-protein activation to changes in gene expression. The rap proteins were of particular interest because they respond to a wide variety of cell surface receptors including GPCRs, and thus could potentially integrate a wide variety of inputs into a homogeneous output. Within the superfamily of low-molecular weight GTPases, the ras GTPases transduce proliferative signals while the closely related rap GTPases are thought to antagonize ras function (51,52). In NIH3T3 cells rap is thought to function as an anti-oncogene, antagonizing the actions of the potent oncogene ras, thus providing a possible explanation why Gs and Gi linked receptors mediate little or no response in cell transformation assays. We reasoned that by linking the effector domain (amino-terminus) of ras to the regulatory domain (carboxyl-terminus) of rap we could convert signals mediated by Gi and Gs-coupled receptors into proliferative outputs. We demonstrated that that conversion of a rap-mediated signal to a ras output is sufficient to enable Gi- and Gs-coupled receptors to transform NIH3T3 cells thus validating this hypothesis. In principle, ras/rap based chimeras could be useful for enabling other gene families as well. As rap is known to respond to a variety of other types of cell surface receptors including tyrosine-kinase linked receptors (53,54), cytokine receptors (55), antigen receptors (56,57) and integrins (58), the strategies outlined above would be expected to work for these gene families as well. In fact, we have observed that ras/rapAA also augments responses to nuclear receptors (unpubl observations). In addition, although we used R-SAT to demonstrate the utility of ras/rap based chimeras, such chimeras could also be used to link GPCRs, and other gene families that activate rap, to activation of reporter constructs responsive to ras-activated signal transduction cascades.

Besides redirecting heterogeneous inputs to a homogeneous output, we were interested in amplifying signal output to increase the signal to noise ratio of the functional assays. The adenylyl cyclase gene family was of interest because these proteins rather than the heterotrimeric G-proteins are thought to be rate limiting in amplification of GPCR-mediated signals (59). Type II adenylyl cyclase was of particular interest because it can respond to Gq- Gi- and Gs-derived signals (39) and we have observed that ACII has very high intrinsic activity relative to other cyclase subtypes (Burstein unpublished observation). Indeed, we observed that overexpression of ACII augmented ras/rap dependent responses although unlike the ras/rap chimeras, ACII itself did not enable significant responses to GPCRs. Thus the role of adenylyl cyclase in enabling functional responses is more as an amplifier and is qualitatively different than that of the ras/rap chimeras, which redirect signals and function as enablers. Besides adenylyl cyclase, we have found that other genes can perform a similar amplifying function. For example the ras-related GTPase rac is required by Gq-coupled receptors to stimulate cellular proliferation (32). We have found that overexpression of rac also augments ras/rap dependent responses to Gs- and Gi-linked GPCRs in R-SAT and can be used in conjunction with ras/rapAA and other genes as part of a helper gene cocktail to enable robust functional responses (see **Figure 5**).

In conclusion, we have demonstrated that chimeras based upon the monomeric GTPases ras and rap, alone or in combination with other genes, enable robust pharmacological responses to a wide variety of GPCRs to be measured in a homogeneous, single configuration. In the context of the R-SAT functional platform, the format described above is well suited to high-throughput applications for the GPCRs superfamily and is ideally suited to the study of orphan GPCRs. Based on the principles described in this paper, it should be possible to configure R-SAT for a much wider array of gene families than previously appreciated. Although preferred embodiments utilize R-SAT technology, other assay formats besides R-SAT can be used to practice the disclosed invention.

Experimental Methods

*Cell culture.* NIH 3T3 cells were incubated at 37 °C in a humidified atmosphere (5% CO2) in Dulbecco's modified Eagles medium supplemented with 4500 mg/l glucose, 4 nM L-glutamine, 50 U/ml penicillin G, 50 U/ml streptomycin (A.B.I.) and 10 % calf serum (Sigma). Pertussis toxin was from Calbiochem.

*Constructs.* All receptors used in the examples below were cloned using polymerase chain reaction using pfu Turbo and the following primer pairs: A Pst1 - KpnI PCR fragment encoding amino acids 61-184 of rap1B together with a Xho I-Pst I PCR fragment encoding amino acids 1-60 of ras (33) was ligated to KpnI - XhoI digested pBluescript. The resulting ras-raplB fragment was then excised with KpnI - NotI, subcloned into pCI (Promega), excised from pCI with MluI - NotI, and subcloned into pSI (Promega). Amino acids Ser 179 and Ser 180 were mutated to alanines using the Quickchange mutagenesis kit (Stratagene) to create the construct used in these studies. All clones were sequence verified. Adenylyl cyclase Type II has been described (34, generous gift of Dr. P. Ram).

*Functional assays.* Receptor Selection and Amplification Technology (R-SAT™) assays were performed as follows: Cells were plated one day before transfection using $2 \times 10^6$ cells in 20 ml of media per 15 cm plate, $2 \times 10^5$ cells in 2 ml of media per 6-well plate, or $7.5 \times 10^3$ cells in 0.1 ml of media per well of a 96-well plate. Cells were transiently transfected using Superfect (Qiagen) according to the manufacturers instructions using 1-20 ng/well of receptor DNA per well of a 96-well plate, 20 ng/well ras/rap chimera, 2 ng/well adenylyl cyclase Type II, and 30 ng/well pSI-β-galactosidase (Promega, Madison, WI) and proportionately more or less for bigger dishes respectively. One day after transfection media was changed and cells were combined with ligands in DMEM supplemented with 2% cyto-SF3 synthetic supplement (Kemp Laboratories) instead of calf serum to a final volume of 200 ul/well. For 15 cm plates cells were trypsinized and aliquoted into the wells of a 96-well plate (100 ul/well) or frozen in aliquots for future use and then subsequently combined with ligands as described above. One 15-cm2 plate yields enough cells for two 96-well or 384-well plates. After five days in culture β-galactosidase levels were measured essentially as described (35). The media were aspirated from the wells and the cells rinsed with phosphate buffered saline (PBS), pH=7.4. 200 ul of PBS with 3.5 mM o-nitrophenyl-β-D-galactopyranoside and 0.5% nonidet P-40 (both Sigma, St. Louis, MO) was added to each well and the 96-well plates were incubated at room temperature. After 3 hr the plates were read at 420 nm on a plate-reader (Bio-Tek EL 310 or Molecular Devices). Dose response data from R-SAT assays were fit to the equation:

$$R = D + (A - D) / (1 + (x / c))$$

where A = minimum response, D = maximum response and c = $EC_{50}$ (R = response, x = concentration of ligand). Cyclic AMP assays were performed using the Biotrak assay kit (Amersham) according to the manufacturers instructions.

*PI turnover assay:* Phosphotidyl inositol hydrolysis assays were carried out in tsA cells (a transformed HEK 293 cell line). Briefly, tsA cells were plated one day before transfection using $1.2 \times 10^5$ cells in 0.1 ml of media per well of a 96-well plate. Cells were transiently transfected as described above using 30 ng receptor per well of a 96-well plate. 24-hours post-transfection, the cells were labeled for 18h in culture medium (0.1ml/well) containing 2uCi/ml of myo-[2-$^3$H] inositol (21Ci/mmol, Perkin-Elmer Life Sciences, Boston, MA). The medium was removed and replaced with Hanks balanced salt solution containing 1mM CaCl2, 1mM MgCl2, 20mM LiCl and 0.1% BSA. The cells were then incubated with ligands for 45min at 37°C. The reaction was stopped using 150ul/well ice-cold 20mM formic acid. Separation of total [$^3$H] inositol phosphates was carried out by ion-exchange chromatography on 1ml-minicolumns loaded with 200ul of a 50% suspension of AG 1-X8 resin (200-400 mesh, Formate form, Bio-Rad, Hercules, CA). Total [$^3$H]-inositol phosphates were counted on LS 6500 Multi-Purpose Scintillation Counter (Beckman Coulter, Fullerton, CA).

*Intracellular cyclic AMP assay:* HEK293 were plated one day before transfection using $1 \times 10^5$ in 0.1 ml of media per well of a 96-well plate. Cells were transiently transfected as described above with using 20 ng receptor per well of a 96-well plate. At 18-20h post-transfection, the medium was removed and the cells were incubated overnight with 200ul/well of 2.0% cyto-sf3/0.5% fetal serum/1% PSG/DMEM. Approximately 40-44 hours post-transfection, medium was replaced with serum-free DMEM/0.1% BSA containing 0.45mM IBMX for 30min (0.1ml/well), then ligands were added for an additional 15 min. The reactions were stopped by exchanging the buffer with 200ul/well of diluted lysis reagent B (Amersham) and agitating the plates for 10 min. The assay plates were either stored at -80C or processed immediately using the cAMP enzyme immunoassay (EIA) assay kit (Amersham Pharmacia Biotech UK, Buckinghamshire, England) according to the manufacturers instructions.

Example 1

This example describes and validates the construction and operation of a reporter chimera and a mutant reporter chimera each comprised of ras sequence fused to rap sequence.

The rap GTPases integrate signaling inputs from Gq, Gs and Gi linked GPCRs (23-29) and ras GTPases stimulate cellular growth in most cell types, thus in principle, ras/rap chimeras which respond to rap inputs, but which mediate ras outputs would link Gq, Gs and Gi derived signals to cellular proliferation. A diagram of this scheme is shown in **Figure 1**. Previously it was shown that certain chimeras between ras and rap retained the ability to transform NIH3T3 cells (33), but in that study only GTPase deficient (deregulated) mutants were used, thus only the effector functions, but none of the regulatory functions of these chimeras were defined. In agreement with those earlier findings, we found that expression of the GTPase impaired mutant forms (derived from viral-ras, denoted v-) of ras, or a chimera between ras and rap1A strongly stimulated cellular proliferation whereas the wild type versions did not (**Figure 2A**). To determine if non-GTPase impaired ras/rap chimeras could stimulate cellular growth, we constructed a series of chimeras containing amino acids 1-60 of c-ras with amino acids 61-184 of rap1B or a mutant form of rap1B in which the consensus site of phosphorylation by PKA (Ser179 and Ser180, see 36), were mutated to alanines (see **Figure 1**) and co-expressed these chimeras with exchange factors known to activate rap. Both EPAC and C3G, which are rap-selective exchange factors, induced robust responses in R-SAT when co-expressed with ras/rap chimeras (**Figures 2B, 2C**). No significant response was observed to the ras/rap chimeras expressed alone and only modest responses were observed to either exchange factor expressed alone. In addition, EPAC, which is activated by the second messenger cyclic AMP (28,29), was further stimulated by 8-Br cAMP to induce ras/rap-dependent responses (see **Figure 2D**) indicating that regulatory controls were preserved at least 2 steps upstream from ras/rap activation. Thus, these ras/rap chimeras stimulate cellular proliferation in a regulated manner.

To assess the specificity of these interactions, the growth promoting effects of the ras-selective exchange factor SOS2 (37) and the rap-selective exchange factors described above were tested with ras/rapAA or ras. As shown in **Figure 2E**, EPAC and C3G induced significant responses only when co-expressed with ras/rapAA whereas SOS2 induced significant responses only when co-expressed with ras. Thus, the sequence determinants for GEF specificity are contained within the C-terminal portions of ras and rap, starting from the switch II region. These results are consistent with a recent study showing that the switch II region is most critical for guanine nucleotide exchange factor recognition of ras and rap (38). Together these results indicate that the ras/rap chimeras are able to receive specific signaling inputs from a rap signal transduction pathway, and transduce signaling outputs that transform cells with similar efficacy to ras.

Example 2

This example demonstrates the utility of redirecting signaling pathways from non-proliferative to proliferative signals using helper genes.

Given that many GPCRs can activate rap we tested whether or not non-transforming GPCRs could stimulate cellular proliferation through the ras/rap chimeras. As shown in **Figure 3A** and **3B,** the Gs-coupled D1 dopaminergic receptor, which stimulates production of cyclic AMP, and the Gi-coupled 5HT1E serotonergic receptor which selectively couples pertussis toxin sensitive G-proteins were unable to produce responses in R-SAT alone. However when these same receptors were co-transfected with ras/rap1B or ras/rapAA, robust agonist dependent responses were observed in R-SAT. Consistently we observed that responses were higher to the ras/rapAA construct indicating that phosphorylation by PKA was unnecessary. Ligand-binding studies showed that expression of ras/rap did not significantly affect expression of either D1 (2.6 pmol/mg alone vs. 2.4 pmol/mg in the presence of ras/rap) or 5HT1E (1.4 pmol/mg alone vs. 1.3 pmol/mg in the presence of ras/rap). Thus D1 and 5HT1E mediated proliferative responses were most likely due to activation of ras/rap and not because of up-regulation of the receptors.

Example 3

This example demonstrates some enabled receptors utilize Gi.

To evaluate the role of Gi-family G-proteins in mediating responses to D1 and 5HT1E, cellular proliferation was examined in the presence of pertussis toxin. As shown in **Figure 4A**, pertussis toxin completely blocked ras/rap dependent responses to 5HT1E confirming that these responses were transduced through Gi-family G-proteins. In contrast, pertussis toxin did not block, and slightly increased ras/rap dependent responses to D1 confirming that D1 mediates stimulatory responses through pertussis insensitive G-proteins. Similar results were obtained with other Gi-linked receptors (**Figure 4B**).

Example 4

This example demonstrates the use of a particular helper construct and a mixture of helper genes.

We next wished to determine whether other signal transduction components could augment ras/rap dependent activity. Type II adenylyl cyclase (AC2) can be activated both by GαS and beta-gamma subunits released by Gαi (39) and thus could potentially augment Gs and Gi-regulated ras/rap dependent activity. As shown in **Figure 5A**, the response to D1 was significantly increased with the addition of Type II adenylyl cyclase. Similarly, ACII augmented responses to Gi-linked receptors such as D2 (**Figure 5B**). However unlike ras/rapAA, ACII itself did not significantly enable Gi- or Gs-linked receptors, though it did augment ras/rap dependent responses (**Figure 5C-5E**). Therefore, for subsequent studies, both ras/rapAA and ACII were included in the transfections and henceforth will collectively be referred to as 'helper genes'.

Besides ACII, we observed that co-transfection of other genes with ras/rapAA augmented ras/rap dependent responses to receptor stimulation. BarkCT is the carboxy-terminal fragment of beta-adrenergic receptor kinase or GRK2, avidly binds to Gbeta-gamma subunits, and therefore inhibits beta-gamma dependent signal transduction pathways (see 60). We were able to confirm that BarkCT inhibits beta-gamma stimulated cellular proliferation as did Transducin, another known scavenger of beta-gamma subunits (**Figure 5F**). However co-expression of BarkCT with ras/rapAA augmented agonist-dependent responses to D1 and MC4 (**Figure 5G** and **5H**) although co-expression of BarkCT alone with D1 did not.

Rac is a ras-related GTPase that is required for GPCR-mediated stimulation of cellular proliferation in NIH3T3 cells (32). As shown in **Figure 5H,** overexpression of rac with BarkCT and ras/rapAA significantly amplified agonist dependent responses to MC4 demonstrating that overexpression of endogenously expressed genes improves the signal to noise ratio of this functional assay. This result also demonstrates the utility of co-expressing a mixture of 'helper genes' to improve assay performance.

Example 5

This example demonstrates the general utility of the described methods for enabling assays for Gs and Gi-linked GPCRs.

To generalize these findings to other GPCRs, a panel of receptors that are known to robustly stimulate the production of cyclic AMP were tested for the ability to produce functional responses in R-SAT either in the presence or the absence of helper genes. As shown in **Figure 6A**, a panel of GPCRs coupled to Gas (beta2 adrenergic, 5HT7 serotonergic, CRF1, MC4 melanocortin, and IP prostanoid) induced little or no response in the absence of helper genes but induced robust responses in the presence of helper genes. Similarly, a group of receptors known to couple PTX-sensitive, Gαi G-proteins (alpha2C adrenergic, D2 dopaminergic, H3 histaminergic, and SST5 somatostatin, see **Figure 6B**) were tested for the ability to produce functional responses in R-SAT either in the presence or the absence of co-transfected helper genes. As for the Gαs-coupled receptors, all of the Gαi -coupled receptors produced robust responses in the presence but not the absence of helper genes. There were no significant responses to any tested ligand on untransfected cells or cells only transfected with helper genes.

Example 6

This example demonstrates that the described assay of GPCR function does not preclude measuring responses to receptors that do not require helpers.

Besides Gs and Gi-linked receptors, it would be desirable to be able to assay Gq and G12/13-linked receptors using the same assay format, particularly when the coupling preferences of the GPCR being tested are unknown, i.e. orphan receptors. Responses to Gq and G12/13 linked receptors can be measured without needing heterologous expression of accessory proteins, thus we tested whether or not co-expression of ras/rapAA and ACII significantly altered the pharmacological responses to receptors that are known to couple Gq and G12/13 proteins such as m1, m5, alphalb, H1, CCKa and PAR2. As shown in **Figure 6C**, co-expression of helper genes did not significantly alter the pharmacological responses to these receptors. Thus through the use of the helper genes described above, one can configure a single assay format compatible with most GPCRs, a particularly desirable property for the functional evaluation of orphan GPCRs.

Example 7

This example compares results to data from other functional assays.

We then compared the observed pharmacological responses determined using R-SAT with data derived from second messenger assays. As shown in **Figure 10**, the $EC_{50}$s for cyclic AMP production by receptors known to couple Gas were in good agreement with the $EC_{50}$s obtained in R-SAT. A slight increase in potency in R-SAT compared to cyclic AMP assays was observed for most of the tested receptors indicating that R-SAT was slightly more sensitive than cyclic AMP measurements. Likewise, for the Gαi coupled receptors evaluated there was good correlation between the $EC_{50}$'s obtained from cyclase inhibition assays and the $EC_{50}$'s obtained in R-SAT. Finally, the ligand potencies at Gq-G12/13-coupled receptors were nearly the same in phosphatidyl inositol hydrolysis assays compared to R-SAT either with or without helper genes. Thus the pharmacology observed for helper gene enabled responses is predictive of the pharmacology observed for commonly used second messenger assays.

Example 8

This example demonstrates the general utility of the described invention with a large number of receptors which do or do not require helper genes.

To assess the general utility of helper genes for enabling detection of functional responses in a homogeneous format, a large array of GPCRs encompassing Gs, Gi, Gq and G12/13 linked GPCRs were tested in R-SAT, all using the helper gene assay format described above. These receptors utilize small molecules, lipids, peptides, and ions for natural ligands, and are classified as family A, B and C type GPCRs. We observed that 90% of the tested receptors were able to mediate potent functional responses with a signal to noise ratio of at least 3 fold using the assay format described above (see Table 1).

**Table 1.**

| G-pro | Receptor | Fold | pEC$_{50}$ | G-pro | Receptor | Fold | pEC$_{50}$ | G-pro | Receptor | Fold | pEC$_{50}$ |
|---|---|---|---|---|---|---|---|---|---|---|---|
| Gi | 5ht1A | 6.9 | 7.4 | Gq | 5ht2A | 3.1 | 9.1 | Gs | 5HT4A | 3.3 | 6.0 |
| Gi | 5ht1B | 2.5 | 8.8 | Gq | 5ht2B | 3.0 | 6.4 | Gs | 5ht6 | 2.6 | 5.4 |
| Gi | 5htlD | 1.5 | 10.7 | Gq | alpha1a/C | 5.1 | 6.8 | Gs | 5ht7A | 4.5 | 9.8 |
| Gi | 5ht1E | 4.3 | 7.7 | Gq | alpha1b | 5.1 | 6.9 | Gs | A2a | 6.9 | 8.3 |
| Gi | 5ht1F | 4.0 | 8.0 | Gq | AT1 | 1.9 | 8.8 | Gs | A2b | 5.1 | 8.1 |
| Gi | A1 | 5.5 | 7.8 | Gq | BK1 | 12.7 | 5.9 | Gs | beta1 | 7.5 | 5.7 |
| Gi | A3 | 3.3 | 7.5 | Gq | BK2 | 4.3 | 6.3 | Gs | beta2 | 4.2 | 8.6 |
| Gi | alpha2a | 7.0 | 7.6 | Gq | CasR wt | 7.0 | 2.3 | Gs | beta3 | 4.5 | 6.9 |
| Gi | alpha2b | 8.6 | 6.8 | Gq | CCKa | 11.0 | 9.6 | Gs | Calcitonin | 4.6 | 9.8 |
| Gi | alpha2C | 6.7 | 7.4 | Gq | CCKb | 30.1 | 8.6 | Gs | CRF1 | 6.7 | 4.4 |
| Gi | CB1 | 7.3 | 7.8 | Gq | ETA | 9.3 | 11.9 | Gs | D1 | 6.8 | 7.5 |
| Gi | CH11-1 | 2.8 | 6.9 | Gq | ETB | 14.8 | 10.3 | Gs | D5 | 4.5 | 7.4 |
| Gi | CH11-5 | 7.3 | 5.8 | Gq | FP | 10.7 | 8.7 | Gs | DP | 2.3 | 11.4 |
| Gi | CX3CR1 | 2.5 | 10.2 | Gq | GHSR1A | 2.1 | 7.7 | Gs | EP2 | 3.2 | 8.7 |
| Gi | D2 | 7.3 | 8.7 | Gq | H1 | 8.8 | 7.3 | Gs | EP4 | 3.3 | 8.6 |
| Gi | D3 | 2.5 | 8.9 | Gq | M1 | 5.5 | 6.4 | Gs | GLP-1 | 3.5 | 7.4 |
| Gi | EP3"D" | 2.8 | 7.9 | Gq | M3 | 11.1 | 6.7 | Gs | Glucagon | 3.3 | 9.6 |
| Gi | GABAb | 3.8 | 7.1 | Gq | M5 | 12.7 | 6.4 | Gs | H2 | 3.2 | 7.7 |
| Gi | H3 | 2.4 | 7.1 | Gq | NK1 | 2.5 | 7.7 | Gs | IP | 7.4 | 0.0 |
| Gi | H4 | 1.6 | 7.9 | Gq | NK2 | 3.5 | 7.9 | Gs | MC4 | 6.4 | 9.8 |
| Gi | Kappa opioid | 3.5 | 7.8 | Gq | NK3 | 3.8 | 7.6 | Gs | PAF | 12.1 | 10.5 |
| Gi | M2 | 4.1 | 6.8 | Gq | PAR1 | 14.0 | 4.9 | Gs | PTH1R | 5.0 | 8.6 |
| Gi | M4 | 7.9 | 5.8 | Gq | PAR2 | 12.0 | 4.5 | Gs | secretin | 4.0 | 9.1 |
| Gi | MT-1 | 3.1 | 7.2 | Gq | PAR4 | 7.8 | 2.9 | Gs | V2 | 3.5 | 10.2 |
| Gi | MT-2 | 3.7 | 7.2 | Gq | TP | 3.8 | 8.5 | Gs | VIP1 | 2.2 | 10.2 |
| Gi | Mu opioid | 2.9 | 7.2 | Gq | urotensin-II | 2.7 | 9.5 | Gs | VIP2 | 5.5 | 8.3 |
| Gi | NPFF2B | 8.6 | 5.2 | Gq | V1A | 9.7 | 9.4 | **Total:** | | **26** | **24** |
| Gi | NPY1 | 2.8 | 9.5 | Gq | V1B | 9.4 | 9.3 | | | | |
| Gi | NPY2 | 3.1 | 9.0 | **Total:** | | **28** | **26** | | | | |
| Gi | NPY5 | 2.6 | 7.2 | | | | | | | | |
| Gi | ORL-1 | 6.3 | 6.7 | | | | | | | | |
| Gi | SST3 | 2.7 | 9.0 | | | | | | | | |

EP 1 692 165 B1

(continued)

| G-pro | Receptor | Fold | pEC$_{50}$ | G-pro | Receptor | Fold | pEC$_{50}$ | G-pro | Receptor | Fold | pEC$_{50}$ |
|---|---|---|---|---|---|---|---|---|---|---|---|
| Gi | SST-2 | 326 | 7.7 | | | | | | | | |
| Gi | SST-5 | 23 | 6.8 | | | | | | | | |
| **Total:** | | **34** | **32** | | | | | | | | |

**Shown are the number of receptors tested, and the number that produced a 2.5-fold response or greater**

Example 9

This example demonstrates the use of the disclosed methods for evaluating function of genes with unknown function.

Given the absence of available ligands, it is necessary to measure constitutive responses to functionally evaluate orphan GPCRs. Previously we showed that constitutive activity of Gq-linked GPCRs could be induced by co-expressing the alpha subunit of Gq (40,41). Using the helper genes described in this paper, we reasoned that it should also be possible to detect constitutive responses of Gs- and Gi-linked orphans as well as Gq-linked orphans. GPR3, GPR6, and GPR12 are three highly related orphan GPCRs that couple Gs (42-44). Drr5, MrgD, and MrgX4 are part of another highly related family of orphans known as the mas-like GPCRs (45). To date, little is known about the functional properties of the mas-like GPCRs. To assess the utility of helper genes for the functional characterization of these orphan GPCRs, we expressed each orphan at increasing doses of transfected plasmid, either alone or in the presence of ras/rapAA & ACII or G$\alpha$q.

As shown in **Figure 7A**, the three mas-like orphans displayed dose-dependent constitutive activity whether or not they were co-expressed with helper genes, but were especially robust when co-expressed with G$\alpha$q suggesting they primarily couple G$\alpha$q. This observation was confirmed using phosphatidyl inositol (PI) hydrolysis assays where each receptor constitutively stimulated PI turnover. In the case of GPR3, 6, & 12, robust constitutive activity was only observed in the presence of helper genes indicating they are most likely G$\alpha$i or Gas coupled (Figure 7B). Direct measurement of cyclic AMP accumulation confirmed that GPR3 and GPR12 strongly couple Gas whereas GPR6 was only weakly coupled. In the presence of pertussis toxin, the helper gene dependent response to GPR6 was significantly blunted whereas the responses to GPR3 and GPR12 were hardly affected indicating GPR6 couples G$\alpha$i-type G-proteins (Figure 7C). Direct comparison of GPR3 and GPR12 to the mas-like GPCRs in cyclic AMP assays confirmed the predictions of signaling specificity based upon the R-SAT data (Figure 7D). Thus, functional characterization of orphan GPCRs is feasibly using the strategies outlined in this paper.

Example 10

This example shows how the disclosed methods can be used to screen for candidate molecules with activity against a particular receptor.

To demonstrate the utility of these methods for drug discovery, cells were transfected with the beta2 receptor, 5HT7 receptor, and the secretin receptor and a mixture of ras/rapAA, ACII, BarkCT, rac, and beta-galactosidase and used to screen a library of 210,000 compounds for agonist activity at these receptors. Using these methods we successfully identified 8 compounds that were agonists at the beta2 receptor; a representative plate with 2 hits is shown in Figure 8. Also shown is an example of profiling one of the hits' against other receptor subtypes; this compound was also a potent beta1 agonist but had no activity at the beta3, D1, D2 or D3 receptor subtypes. In all cases the activity of these compounds was only apparent on cells expressing both the receptor and helper genes described above; no activity was seen in cells expressing the receptor alone, the helpers alone, or other receptor subtypes and the helpers. These results demonstrate the utility of this 'helper gene' strategy for drug discovery.

References

All of the references cited below are incorporated herein by reference in their entirety:

1. International Human Genome Sequencing Consortium Initial sequencing and analysis of the human genome Nature 409:860-921

2. Kenakin T. The measurement of efficacy in the drug discovery agonist selection process. J. Pharmacol. Toxicol. Meth. (2000) 42:177-187.

3. Stables J, Green A, Marshall F, Fraser N, Knight E, Sautel M, Milligan G, Lee M, Rees S. A bioluminescent assay for agonist activity at potentially any G-protein-coupled receptor. Anal Biochem 1997 Oct 1;252(1):115-26

4. Sullivan E, Tucker EM, Dale IL. Measurement of [Ca2+] using the Fluorometric Imaging Plate Reader (FLIPR). Methods Mol Biol 1999; 114:125-33

5. Porter RH, Benwell KR, Lamb H, Malcolm CS, Allen NH, Revell DF, Adams DR, Sheardown MJ Functional characterization of agonists at recombinant human 5-HT2A, 5-HT2B and 5-HT2C receptors in CHO-K1 cells. Br J Pharmacol 1999 Sep; 128(1):13-20

6. Salomon Y. Cellular responsiveness to hormones and neurotransmitters: conversion of [3H]adenine to [3H] cAMP in cell monolayers, cell suspensions, and tissue slices. Methods Enzymol. (1991) 195:22-28

7. Chen W., Shields T.S., Stork P.J.S., and Cone R.D. A colorimetric assay for measuring activation of Gs- and Gq-coupled signaling pathways. Anal Biochem. (1995) 226:349-54.

8. Potenza M.N., Graminski G.F., and Lerner M.R. A method for evaluating the effects of ligands upon Gs protein-coupled receptors using a recombinant melanophore-based bioassay. Anal Biochem. (1992) 206:315-22.

9. Brauner-Osborne H., and Brann M.R. Pharmacology of muscarinic acetylcholine receptor subtypes (m1-m5): high throughput assays in mammalian cells. Eur J Pharmacol. (1996) 295:93-102.

10. Burstein ES, Brauner-Osborne H, Spalding TA, Conklin BR, Brann MR Interactions of muscarinic receptors with the heterotrimeric G proteins Gq and G12: transduction of proliferative signals. J Neurochem. (1997) 68:525-33.

11. Conklin BR, Farfel Z, Lustig KD, Julius D, Bourne HR. Substitution of three amino acids switches receptor specificity of Gq alpha to that of Gi alpha.Nature 1993 May 20;363(6426):274-6

12. Conklin BR, Herzmark P, Ishida S, Voyno-Yasenetskaya TA, Sun Y, Farfel Z, Bourne HR Carboxyl-terminal mutations of Gq alpha and Gs alpha that alter the fidelity of receptor activation. Mol Pharmacol. (1996) 50:885-90.

13. Amatruda TT 3rd, Steele DA, Slepak VZ, Simon MI. G alpha 16, a G protein alpha subunit specifically expressed in hematopoietic cells. Proc Nat1 Acad Sci U S A 1991 Jul 1;88(13):5587-91

14. Offermanns S, Simon MI. G alpha 15 and G alpha 16 couple a wide variety of receptors to phospholipase C. J Biol Chem 1995 Jun 23;270(25):15175-80

15. Simon MI, Strathmann MP, Gautam N. Diversity of G proteins in signal transduction. Science 1991 May 10;252(5007):802-8

16. Strathmann MP, Simon MI. G alpha 12 and G alpha 13 subunits define a fourth class of G protein alpha subunits. Proc Natl Acad Sci U S A 1991 Jul 1;88(13):5582-6

17. Gomeza J, Mary S, Brabet I, Parmentier ML, Restituito S, Bockaert J, Pin JP. Coupling of metabotropic glutamate receptors 2 and 4 to G alpha 15, G alpha 16, and chimeric G alpha q/i proteins: characterization of new antagonists. Mol Pharmacol 1996 Oct;50(4):923-30

18. Lee JW, Joshi S, Chan JS, Wong YH Differential coupling of mu-, delta-, and kappa-opioid receptors to G alpha16-mediated stimulation of phospholipase C. J Neurochem. (1998) 70:2203-11.

19. Kostenis E. Is Galpha16 the optimal tool for fishing ligands of orphan G-protein-coupled receptors? Trends Pharmacol Sci 2001 Nov;22(11):560-4

20. Bokoch GM. Interplay between Ras-related and heterotrimeric GTP binding proteins: lifestyles of the BIG and little. FASEB J 1996 Sep; 10(11):1290-5

21. Quilliam LA, Rebhun JF, Castro AF. A growing family of guanine nucleotide exchange factors is responsible for activation of Ras-family GTPases. Prog Nucleic Acid Res Mol Biol. 2002;71:391-444.

22. Cherfils J, Chardin P. GEFs: structural basis for their activation of small GTP-binding proteins. Trends Biochem Sci. 1999 Aug;24(8):306-11.

23. Seidel MG, Klinger M, Freissmuth M, Holler C. Activation of mitogen-activated protein kinase by the A (2A)-adenosine receptor via a rap1-dependent and via a p21(ras)-dependent pathway. J Biol Chem 1999 Sep 3;274 (36):25833-41

24. Schmitt, J. M., and P. J. S. Stork. 2000. Beta-adrenergic receptor activates extracellular regulated kinases (ERKs) via the small G protein Rap1 and the serine/threonine kinase B-Raf. J. Biol. Chem. 275:25342-25350

25. Lova P, Paganini S, Sinigaglia F, Balduini C, Torti M. A Gi-dependent pathway is required for activation of the small GTPase RaplB in human platelets. J Biol Chem. 2002 Apr 5;277(14):12009-15.

26. Woulfe D, Jiang H, Mortensen R, Yang J, Brass LF. Activation of RaplB by G(i) family members in platelets. J Biol Chem. 2002; 277(26):23382-90.

27. Guo FF, Kumahara E, Saffen D. A CalDAG-GEFI/Rap1/B-Raf cassette couples M(1) muscarinic acetylcholine receptors to the activation of ERK1/2. J Biol Chem 276:25568-25581 (2001).

28. de Rooij J, Zwartkruis FJT, Verheijen MHG, Cool RH, Nijman SMB, Wittinghofer A & Bos JL Epac is a Rap1 guanine-nuecleotide-exchange factor directly activated by cyclic AMP. Nature 396:474-477 (1998).

29. Kawasaki H, Springett GM, Mochizuki N, Toki S, Nakaya M, Matsuda M, Housman DE, & Graybiel AM. A family of cAMP-binding proteins that directly activate Rap1. Science 282:2275-2279 (1998).

30. Messier TL, Dorman CM, Brauner-Osborne H, Eubanks D, and Brann MR High throughput assays of cloned adrenergic, muscarinic, neurokinin, and neurotrophin receptors in living mammalian cells. Pharmacol Toxicol (1995) 76: 308-11

31. Burstein E.S., Spalding T.A., Hill-Eubanks D., and Brann M.R. Structure-function of muscarinic receptor coupling to G proteins. Random saturation mutagenesis identifies a critical determinant of receptor affinity for G proteins. J Biol Chem. (1995) 270:3141-6.

32. Burstein ES, Hesterberg DJ, Gutkind JS, Brann MR, Currier EA, Messier TL The ras-related GTPase rac1 regulates a proliferative pathway selectively utilized by G-protein coupled receptors. Oncogene (1998) 24:1617-23.

33. Zhang K, Noda M, Vass WC, Papageorge AG, Lowy DR. Identification of small clusters of divergent amino acids that mediate the opposing effects of ras and Krev-1. Science. (1990) 249:162-5.

34. Smit MJ, Verzijl D, Iyengar R Identity of adenylyl cyclase isoform determines the rate of cell cycle progression in NIH 3T3 cells. Proc Natl Acad Sci U S A (1998) 95:15084-9.

35. Lim K., and Chae C.-B. (1989) A Simple Assay for DNATransfection by Incubation of the Cells in Culture Dishes with Substrates for □-galactosidase. Bio-Techniques 7:576-579.

36. Altschuler D, Lapetina EG. Mutational analysis of the cAMP-dependent protein kinase-mediated phosphorylation site of Raplb. J Biol Chem. 1993; 268(10):7527-31.

37. Chardin P, Camonis JH, Gale NW, van Aelst L, Schlessinger J, Wigler MH, Bar-Sagi D. Human Sos1: a guanine nucleotide exchange factor for Ras that binds to GRB2. Science 1993 May 28;260(5112):1338-43

38. van den Berghe N, Cool RH, Wittinghofer A. Discriminatory residues in Ras and Rap for guanine nucleotide exchange factor recognition. J Biol Chem. 1999 Apr 16;274(16):11078-85.

39. Lustig KD, Conklin BR, Herzmark P, Taussig R, Bourne HR. Type II adenylylcyclase integrates coincident signals from Gs, Gi, and Gq. J Biol Chem 1993 Jul 5;268(19):13900-5

40. Burstein ES, Spalding TA, Brann MR. Pharmacology of muscarinic receptor subtypes constitutively activated by G proteins. Mol Pharmacol. 1997 Feb;51(2):312-9.

41. Burstein ES, Spalding TA, Brauner-Osborne H, Brann MR. Constitutive activation of muscarinic receptors by the G-protein Gq. FEBS Lett. 1995 Apr 24;363(3):261-3.

42. Song ZH, Modi W, Bonner TI. Molecular cloning and chromosomal localization of human genes encoding three closely related G protein-coupled receptors. Genomics 1995 Jul 20;28(2):347-9

43. Eggerickx D, Denef JF, Labbe O, Hayashi Y, Refetoff S, Vassart G, Parmentier M, Libert F. Molecular cloning of an orphan G-protein-coupled receptor that constitutively activates adenylate cyclase. Biochem J 1995 Aug 1; 309 ( Pt 3):837-43

44. Uhlenbrock K, Gassenhuber H, Kostenis E. Sphingosine 1-phosphate is a ligand of the human gpr3, gpr6 and gpr12 family of constitutively active G protein-coupled receptors. Cell Signal. 2002 Nov;14(11):941.

45. Dong X, Han S, Zylka MJ, Simon MI, Anderson DJ. A diverse family of GPCRs expressed in specific subsets of nociceptive sensory neurons. Cell 2001 Sep 7;106(5):619-32

46. Chan AM, Fleming TP, McGovern ES, Chedid M, Miki T, Aaronson SA. Expression cDNA cloning of a transforming gene encoding the wild-type G alpha 12 gene product. Mol Cell Biol 1993 Feb;13(2):762-8

47. Xu N, Voyno-Yasenetskaya T, Gutkind JS. Potent transforming activity of the G13 alpha subunit defines a novel family of oncogenes. Biochem Biophys Res Commun 1994 Jun 15;201(2):603-9

48. Wu J, Dent P, Jelinek T, Wolfman A, Weber MJ, Sturgill TW Inhibition of the EGF-activated MAP kinase signaling pathway by adenosine 3',5'-monophosphate. Science (1993) 262:1065-9.

49. Cook SJ, McCormick F Inhibition by cAMP of Ras-dependent activation of Raf. Science. (1993) 262:1069-72.

50. Schmitt JM, Stork PJ Cyclic AMP-mediated inhibition of cell growth requires the small G protein Rap1. Mol Cell Biol. (2001) 11:3671-83.

51. Bos JL, de Rooij J, Reedquist KA Rap1 signaling: adhering to new models. Nat Rev Mol Cell Biol. (2001) 2:369-77

52. Kitayama H, Sugimoto Y, Matsuzaki T, Ikawa Y, Noda M A ras-related gene with transformation suppressor activity. Cell. (1989) 56:77-84

53. York RD, Yao H, Dillon T, Ellig CL, Eckert SP, McCleskey EW & Stork PJS Rap1 mediates sustained MAP kinase activation induced by nerve growth factor. Nature 392:622-626 (1998).

54. Wu C, Lai CF, Mobley WC. Nerve growth factor activates persistent Rap1 signaling in endosomes. J Neurosci. 2001 Aug 1;21(15):5406-16

55. M'Rabet L, Coffer P, Zwartkruis F, Franke B, Segal AW, Koenderman L, Bos JL. Activation of the small GTPase rap1 in human neutrophils. Blood. 1998 Sep 15;92(6):2133-40.

56. McLeod SJ, Ingham RJ, Bos JL, Kurosaki T, Gold MR. Activation of the Rap1 GTPase by the B cell antigen receptor. J Biol Chem. 1998 Oct 30; 273(44): 29218-23.

57. Katagiri K, Hattori M, Minato N, Kinashi T. Rap1 functions as a key regulator of T-cell and antigen-presenting cell interactions and modulates T-cell responses. Mol Cell Biol. 2002 Feb; 22(4): 1001-15.

58. Reedquist, K. A., E. Ross, E. A. Koop, R. M. Wolthuis, F. J. Zwartkruis, Y. van Kooyk, M. Salmon, C. D. Buckley, and J. L. Bos. 2000. The Small GTPase, Rap1, mediates CD31-induced integrin adhesion. J. Cell Biol. 148: 1151-1158

59. Ostrom RS, Violin JD, Coleman S, Insel PA Selective enhancement of beta-adrenergic receptor signaling by overexpression of adenylyl cyclase type 6: colocalization of receptor and adenylyl cyclase in caveolae of cardiac myocytes. Mol Pharmacol. (2000) 57:1075-9.

60. Koch WJ, Hawes BE, Allen LF, Lefkowitz RJ. Direct evidence that Gi-coupled receptor stimulation of mitogen-activated protein kinase is mediated by G beta gamma activation of p21ras. Proc Natl Acad Sci U S A. 1994 Dec 20;91(26):12706-10.

**Claims**

1.  A method of determining whether a substance is a ligand for a G-protein coupled receptor (GPCR), comprising:

    (a) providing a cell culture which comprises cells comprising a first nucleic acid which encodes a GPCR which increases or decreases cellular proliferation through a GPCR cascade in response to a ligand, a second recombinant nucleic acid which encodes a marker indicative of the extent of cellular proliferation, and a third recombinant nucleic acid encoding a Ras effector domain and a Rap input domain operably coupled to said Ras effector domain;
    (b) contacting said cell culture with a test compound; and
    (c) determining the level of cellular proliferation.

2.  The method of Claim 1, wherein the Rap sequence does not contain any mutations.

3.  The method of Claim 1, wherein the Rap input domain includes at least one missense mutation.

4.  The method of Claim 3, wherein said at least one missense mutation in said Rap protein prevents phosphorylation of said Rap input domain.

5.  The method of Claim 1, wherein the Ras effector domain is not GTPase-deficient.

6.  The method of Claim 1, wherein said population of cells further comprises a fourth recombinant nucleic acid construct that operably encodes a helper protein other than the helper protein encoded by said third recombinant nucleic acid.

7.  The method of Claim 6, wherein said helper protein functions upstream of the Rap input domain in the G-protein coupled receptor signal transduction cascade.

8.  The method of Claim 6, wherein said helper protein functions downstream of the Rap input domain in the G-protein coupled receptor signal transduction cascade.

9.  The method of Claim 6, wherein said helper protein reduces the signal transduction through G protein coupled receptors other than the recombinant G protein coupled receptor encoded on the first recombinant nucleic acid.

10. The method of Claim 6, wherein said helper protein amplifies the proliferation through the Ras effector domain.

11. The method of Claim 10, wherein said helper protein is a $G\beta\gamma$ scavenger.

12. The method of Claim 11, wherein said $G\beta\gamma$ scavenger is Transducin.

13. The method of Claim 11, wherein said $G\beta\gamma$ scavenger is BarkCT.

14. The method of Claim 9, wherein said helper protein is a fragment of a G protein coupled receptor other than the G protein coupled receptor encoded by said first recombinant nucleic acid.

15. The method of Claim 7, wherein said protein that functions upstream of the Rap input domain is a Go protein.

16. The method of Claim 15, wherein said Go protein is selected from the group consisting of $Ga_s$, $Ga_q$, $Ga_i$ and $Ga_{12/13}$.

17. The method of Claim 7, wherein said helper protein is a guanine nucleotide exchange factor (GEF).

18. The method of Claim 17, wherein said helper protein is EPAC.

19. The method of Claim 17, wherein said helper protein is C3G.

20. The method of Claim 7, wherein said helper protein is a cyclase.

21. The method of Claim 20, wherein said cyclase is an adenylyl cyclase.

**22.** The method of Claim 21, wherein said helper protein is adenylyl cyclase II (ACII).

**23.** The method of Claim 7, wherein said helper protein is a GTPase.

**24.** The method of Claim 23, wherein said GTPase is Rac.

**25.** The method of Claim 1, wherein said G-protein coupled receptor is a Gs-linked G protein coupled receptor.

**26.** The method of Claim 1, wherein said G protein coupled receptor is a Gi linked G protein coupled receptor.

**27.** The method of Claim 1, wherein said G protein coupled receptor is a Gq linked G protein coupled receptor.

**28.** The method of Claim 1, wherein said G protein coupled receptor is a G12/G13 linked protein coupled receptor.

**29.** The method of Claim 1, wherein said step of determining said level of cellular proliferation comprises transfecting said cell culture with a fourth nucleic acid that encodes β-galactosidase and measuring the amount of β-galactosidase activity of said cell culture.

**30.** The method of Claim 1, wherein the step of determining the level of cellular proliferation comprises performing a Receptor Selection and Amplification Technology Assay.

**Patentansprüche**

**1.** Verfahren zum Bestimmen, ob es sich bei einer Substanz um einen Liganden für einen G-Protein-gekoppelten Rezeptor (GPCR) handelt, das folgendes umfaßt:

(a) Bereitstellen einer Zellkultur, die Zellen umfaßt, die eine erste Nukleinsäure umfassen, die für einen GPCR, der die Zellproliferation über eine GPCR-Kaskade in Reaktion auf einen Liganden verstärkt oder verringert, kodiert, eine zweite rekombinante Nukleinsäure, die für einen Marker, der das Ausmaß der Zellproliferation angibt, kodiert, sowie eine dritte rekombinante Nukleinsäure, die für eine Ras-Effektordomäne und eine Rap-Inputdomäne in operativer Verknüpfung mit der Ras-Effektordomäne kodiert;
(b) In-Kontakt-Bringen der Zellkultur mit einer Testverbindung; und
(c) Bestimmen des Ausmaßes der Zellproliferation.

**2.** Verfahren nach Anspruch 1, wobei die Rap-Sequenz keine Mutationen enthält.

**3.** Verfahren nach Anspruch 1, wobei die Rap-Inputdomäne mindestens eine Missense-Mutation beinhaltet.

**4.** Verfahren nach Anspruch 3, wobei die mindestens eine Missense-Mutation in dem Rap-Protein die Phosphorylierung der Rap-Inputdomäne verhindert.

**5.** Verfahren nach Anspruch 1, wobei die Ras-Effektordomäne nicht GTPase-defizient ist.

**6.** Verfahren nach Anspruch 1, wobei die Zellpopulation weiterhin ein viertes rekombinantes Nukleinsäurekonstrukt umfaßt, welches operativ für ein Helferprotein, bei dem es sich nicht um das von der dritten rekombinanten Nukleinsäure kodierte Helferprotein handelt, kodiert.

**7.** Verfahren nach Anspruch 6, wobei das Helferprotein stromaufwärts von der Rap-Inputdomäne in der Signaltransduktionskaskade des G-Protein-gekoppelten Rezeptors agiert.

**8.** Verfahren nach Anspruch 6, wobei das Helferprotein stromabwärts von der Rap-Inputdomäne in der Signaltransduktionskaskade des G-Protein-gekoppelten Rezeptors agiert.

**9.** Verfahren nach Anspruch 6, wobei das Helferprotein die Signaltransduktion durch G-Protein-gekoppelte Rezeptoren, bei denen es sich nicht um den auf der ersten rekombinanten Nukleinsäure kodierten rekombinanten G-Protein-gekoppelten Rezeptor handelt, verringert.

**10.** Verfahren nach Anspruch 6, wobei das Helferprotein die Proliferation durch die Ras-Effektordomäne amplifiziert.

**11.** Verfahren nach Anspruch 10, wobei es sich bei dem Helferprotein um einen Gβγ-Scavenger handelt.

**12.** Verfahren nach Anspruch 11, wobei es sich bei dem Gβγ-Scavenger um Transducin handelt.

**13.** Verfahren nach Anspruch 11, wobei es sich bei dem Gβγ-Scavenger um BarkCT handelt.

**14.** Verfahren nach Anspruch 9, wobei es sich bei dem Helferprotein um ein Fragment eines G-Protein-gekoppelten Rezeptors, bei dem es sich nicht um den von der ersten rekombinanten Nukleinsäure kodierten G-Protein-gekoppelten Rezeptor handelt, handelt.

**15.** Verfahren nach Anspruch 7, wobei dieses Protein, das stromaufwärts von der Rap-Inputdomäne agiert, um ein Go-Protein handelt.

**16.** Verfahren nach Anspruch 15, wobei dieses Go-Protein aus der Gruppe Go$_s$, Go$_q$, Go$_i$ und Go$_{12/13}$ stammt.

**17.** Verfahren nach Anspruch 7, wobei es sich bei diesem Helferprotein um einen Guaninnukleotidaustauschfaktor (GEF) handelt.

**18.** Verfahren nach Anspruch 17, wobei es sich bei dem Helferprotein um EPAC handelt.

**19.** Verfahren nach Anspruch 17, wobei es sich bei dem Helferprotein um C3G handelt.

**20.** Verfahren nach Anspruch 7, wobei es sich bei dem Helferprotein um eine Cyclase handelt.

**21.** Verfahren nach Anspruch 20, wobei es sich bei der Cyclase um eine Adenylylcyclase handelt.

**22.** Verfahren nach Anspruch 21, wobei es sich bei dem Helferprotein um die Adenylylcyclase II (ACII) handelt.

**23.** Verfahren nach Anspruch 7, wobei es sich bei dem Helferprotein um eine GTPase handelt.

**24.** Verfahren nach Anspruch 23, wobei es sich bei der GTPase um Rac handelt.

**25.** Verfahren nach Anspruch 1, wobei es sich bei dem G-Protein-gekoppelten Rezeptor um einen Gsgekoppelten G-Protein-gekoppelten Rezeptor handelt.

**26.** Verfahren nach Anspruch 1, wobei es sich bei dem G-Protein-gekoppelten Rezeptor um einen Gigekoppelten G-Protein-gekoppelten Rezeptor handelt.

**27.** Verfahren nach Anspruch 1, wobei es sich bei dem G-Protein-gekoppelten Rezeptor um einen Gqgekoppelten G-Protein-gekoppelten Rezeptor handelt.

**28.** Verfahren nach Anspruch 1, wobei es sich bei dem G-Protein-gekoppelten Rezeptor um einen G12/13-gekoppelten proteingekoppelten Rezeptor handelt.

**29.** Verfahren nach Anspruch 1, wobei der Schritt des Bestimmens des Ausmaßes der Zellproliferation umfaßt, daß man die Zellkultur mit einer vierten Nukleinsäure, die für β-Galactosidase kodiert, transfiziert und das Ausmaß der β-Galactosidaseaktivität der Zellkultur mißt.

**30.** Verfahren nach Anspruch 1, wobei der Schritt des Bestimmens des Ausmaßes der Zellproliferation umfaßt, daß man einen Rezeptorselektions- und Amplifikationstechnologie-Assay durchführt.

**Revendications**

**1.** Méthode permettant de déterminer si une substance est un ligand d'un récepteur couplé aux protéines G (RCPG), comprenant les étapes consistant à :

(a) obtenir une culture cellulaire qui comprend des cellules comprenant un premier acide nucléique qui code pour un RCPG qui augmente ou diminue la prolifération cellulaire par l'intermédiaire d'une cascade de RCPG en réponse à un ligand, un deuxième acide nucléique recombinant qui code pour un marqueur indiquant l'ampleur de la prolifération cellulaire et un troisième acide nucléique recombinant codant pour un domaine effecteur de Ras et un domaine d'entrée de Rap couplé de manière opérationnelle audit domaine effecteur de Ras ;
(b) mettre ladite culture cellulaire en contact avec un composé à l'essai ; et
(c) déterminer le niveau de prolifération cellulaire.

2. Méthode selon la revendication 1, selon laquelle la séquence de Rap ne contient aucune mutation.

3. Méthode selon la revendication 1, selon laquelle le domaine d'entrée de Rap inclut au moins une mutation faux-sens.

4. Méthode selon la revendication 3, selon laquelle ladite ou lesdites mutations faux-sens dans ladite protéine Rap empêchent la phosphorylation dudit domaine d'entrée de Rap.

5. Méthode selon la revendication 1, selon laquelle le domaine effecteur de Ras n'est pas déficient en activité GTPase.

6. Méthode selon la revendication 1, selon laquelle ladite population de cellules comprend en outre une quatrième construction d'acide nucléique recombinante qui code de manière opérationnelle pour une protéine auxiliaire autre que la protéine auxiliaire codée par ledit troisième acide nucléique recombinant.

7. Méthode selon la revendication 6, selon laquelle ladite protéine auxiliaire fonctionne en amont du domaine d'entrée de Rap dans la cascade de transduction du signal du récepteur couplé aux protéines G.

8. Méthode selon la revendication 6, selon laquelle ladite protéine auxiliaire fonctionne en aval du domaine d'entrée de Rap dans la cascade de transduction du signal du récepteur couplé aux protéines G.

9. Méthode selon la revendication 6, selon laquelle ladite protéine auxiliaire réduit la transduction du signal par l'intermédiaire de récepteurs couplés aux protéines G autres que le récepteur couplé aux protéines G recombinant codé par le premier acide nucléique recombinant.

10. Méthode selon la revendication 6, selon laquelle ladite protéine auxiliaire amplifie la prolifération par l'intermédiaire du domaine effecteur de Ras.

11. Méthode selon la revendication 10, selon laquelle ladite protéine auxiliaire est un scavenger de $G\beta\gamma$.

12. Méthode selon la revendication 11, selon laquelle ledit scavenger de $G\beta\gamma$ est la transducine.

13. Méthode selon la revendication 11, selon laquelle ledit scavenger de $G\beta\gamma$ est BarkCT.

14. Méthode selon la revendication 9, selon laquelle ladite protéine auxiliaire est un fragment d'un récepteur couplé aux protéines G autre que le récepteur couplé aux protéines G codé par ledit premier acide nucléique recombinant.

15. Méthode selon la revendication 7, selon laquelle ladite protéine qui fonctionne en amont du domaine d'entrée de Rap est une protéine $G\alpha$.

16. Méthode selon la revendication 15, selon laquelle ladite protéine $G\alpha$ est choisie dans le groupe constitué de $G\alpha_s$, de $G\alpha_q$, de $G\alpha_i$ et de $G\alpha_{12/13}$.

17. Méthode selon la revendication 7, selon laquelle ladite protéine auxiliaire est un facteur d'échange de nucléotide guanine (GEF).

18. Méthode selon la revendication 17, selon laquelle ladite protéine auxiliaire est EPAC.

19. Méthode selon la revendication 17, selon laquelle ladite protéine auxiliaire est C3G.

20. Méthode selon la revendication 7, selon laquelle ladite protéine auxiliaire est une cyclase.

**21.** Méthode selon la revendication 20, selon laquelle ladite cyclase est une adénylyl cyclase.

**22.** Méthode selon la revendication 21, selon laquelle ladite protéine auxiliaire est l'adénylyl cyclase II (ACII).

**23.** Méthode selon la revendication 7, selon laquelle ladite protéine auxiliaire est une GTPase.

**24.** Méthode selon la revendication 23, selon laquelle ladite GTPase est Rac.

**25.** Méthode selon la revendication 1, selon laquelle ledit récepteur couplé aux protéines G est un récepteur couplé aux protéines G lié à $G_s$.

**26.** Méthode selon la revendication 1, selon laquelle ledit récepteur couplé aux protéines G est un récepteur couplé aux protéines G lié à $G_i$.

**27.** Méthode selon la revendication 1, selon laquelle ledit récepteur couplé aux protéines G est un récepteur couplé aux protéines G lié à $G_q$.

**28.** Méthode selon la revendication 1, selon laquelle ledit récepteur couplé aux protéines G est un récepteur couplé aux protéines G lié à $G_{12/13}$.

**29.** Méthode selon la revendication 1, selon laquelle ladite étape consistant à déterminer ledit niveau de prolifération cellulaire comprend la transfection de ladite culture cellulaire avec un quatrième acide nucléique qui code pour une β-galactosidase et la mesure de la quantité d'activité β-galactosidase de ladite culture cellulaire.

**30.** Méthode selon la revendication 1, selon laquelle ladite étape consistant à déterminer le niveau de prolifération cellulaire comprend la réalisation d'un dosage basé sur la technologie de sélection et d'amplification du récepteur (« Receptor Selection and Amplification Technology »).

# Fig 1: Construction/function of ras/rap chimeras

Signal Input

Effector
Domain

Regulatory
Domain

Rap:     N ▦▦▦▦▦▦▦▦▦▦▦SS▦ C

Ras:     N ████████████████ C

Ras/rap:    N ████████▦▦▦▦SS▦ C

Ras/rapAA:  N ████████▦▦▦▦AA▦ C

Signal Output

# Figure 2A. Activation of cellular proliferation by ras/rap chimera.

# Figure 2B, 2C. Rap selective exchange factors activate cellular proliferation in a ras/rap chimera dependent manner

# Figure 2D: Activation of EPAC by 8-Br cAMP

# Figure 2E. Rap exchange factors are selective for ras-rap chimeras.

# Figure 3. Non-transforming G-protein coupled receptors activate cellular proliferation in a ras/rap dependent manner

A) D1

B) 5HT1E

# Figure 4. Pertussis toxin sensitivity of ras/rap dependent proliferative responses to G-protein coupled receptors.

A) D1

B) 5HT1E

# Figure 4C:  Gi-coupled receptors enabled by Ras/rap are blocked by PTX

# Figure 5. Adenylyl cyclase augments ras-rap dependant responses to D1 and D2

## A) D1

Legend: ● Ras/rap+AC2, ○ Ras/rap

Y-axis: Response (%), X-axis: [SKF81297]

## B) D2

Legend: ● Ras/rap+AC2, ○ Ras/rap

Y-axis: Response (%), X-axis: [Quinpirole]

# Figure 5C & 5D: AC2 augments enabling effect of Ras/rap: Gi-linked receptors

## D2

Legend:
- ● Ras/rap & AC2
- ○ Ras/rap
- ■ AC2
- □ control

Response (%): 200, 100, 0

x-axis: -13 -12 -11 -10 -9 -8 -7 -6

[Pergolide]

## Alpha2C

Legend:
- ● Ras/rap & AC2
- ○ Ras/rap
- ■ AC2
- □ control

Response (%): 200, 100, 0

x-axis: -12 -11 -10 -9 -8 -7 -6 -5

[UK14304]

# Figure 5E: AC2 augments enabling effect of Ras/rap: Gs-linked receptors

MC4

[DNP-α-MSH]

# Figure 5F: Suppression of beta-gamma signaling by BarkCT

# Figure 5G: Synergy of helper genes

# Figure 5H: Synergy of helper genes

# Figure 6A. Gs-coupled receptors enabled with Ras/rapAC2 for cell proliferation assay

EP 1 692 165 B1

Figure 6B. Gi-coupled receptors enabled with Ras/RapAC2 for cell proliferation assay

# Figure 6C: Group of Gq receptors enabled +/- Ras/RapAC2 for cell proliferation assay

Figure 7A: Gq-selective orphans

Drr5

alone
Gq
r/rAC2

Response (A.U.)

500 ng   50 ng   control

MrgD

alone
Gq
r/rAC2

Response (A.U.)

500 ng   50 ng   control

MrgX4

alone
Gq
r/rAC2

Response (A.U.)

500 ng   50 ng   control

PI Hydrolysis

Response (dpm)

MrgX4   Drr5   MrgD   control

# Figure 7B: Gs-selective orphans

GPR12 — Response (A.U.) vs 500 ng, 50 ng, control; legend: alone, Gq, r/rAC2

GPR6 — Response (A.U.) vs 500 ng, 50 ng, control; legend: alone, Gq, r/rAC2

GPR3 — Response (A.U.) vs 500 ng, 50 ng, control; legend: alone, Gq, r/rAC2

cAMP — cyclic AMP (fmol) vs GPR12, GPR3, GPR6, control

EP 1 692 165 B1

# Figure 7C: Orphan specificity for the Gi-pathway

## PTX sensitivity

*significant repression

EP 1 692 165 B1

# Figure 7D: Orphan specificity for the Gs-pathway

## cAMP

# Figure 8. HTS screening using helper genes: proof of concept

210,000 compound library
HTS screen at beta2 receptor

Plate AX0019W Chart

Hits!    Controls

1 2 3 4 5 6 7 8 9 10 11 12 13 14 15 16 17 18 19 20 21 22 23 24

HTS profile

Response (A.U.)

-10 -9 -8 -7 -6 -5
[Active Hit]

■ beta1
● beta2
□ beta3
○ D1
◆ D2
▲ D3

Potent, selective leads!

EP 1 692 165 B1

# Figure 9. Homogeneous detection of GPCR signaling using ras/rap chimeras

EP 1 692 165 B1

# Figure 10: R-SAT vs. 2$^{nd}$ messenger assays

Gs receptors:

| Receptor | R-SAT | cAMP stim | ratio |
|---|---|---|---|
| 5HT7a | 10.1 | 9.4 | 5 |
| Beta2 | 8.8 | 8.1 | 5 |
| CRF1 | 9.0 | 9.1 | 1 |
| D1 | 7.3 | 7.6 | 0.5 |
| IP | 7.4 | 7.0 | 3 |
| MC4 | 9.8 | 9.1 | 6 |

Gi receptors:

| Receptor | R-SAT | cAMP inhib | ratio |
|---|---|---|---|
| M4 | 6.3 | 6.4 | 1 |
| 5HT1E | 7.4 | 7.6 | 1 |
| D2 | 8.2 | 7.7 | 3 |
| alpha2C | 6.9 | 7.5 | 0.3 |
| SSTR5 | 8.0 | 8.7 | 0.2 |
| NPFF2b | 5.2 | nd | - |

Gq receptors:

| Receptor | R-SAT | R-SAT & helpers | PI hydr | Ratio h/no-h | Ratio PI/RSAT |
|---|---|---|---|---|---|
| Alpha1B | 6.2 | 6.8 | 6.9 | 1.1 | 1.1 |
| CCKa | 8.3 | 8.5 | 8.2 | 1 | 1.0 |
| H1 | 6.9 | 7.0 | 7.4 | 1 | 1.1 |
| m1 | 6.6 | 6.7 | 5.9 | 1 | 0.9 |
| m5 | 6.3 | 6.0 | 5.9 | 1 | 0.9 |
| PAR2 | 4.6 | 4.5 | 5.0 | 1 | 1.1 |

EP 1 692 165 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5707798 A
- US 6358698 B
- US 5955281 A
- US 5912132 A

### Non-patent literature cited in the description

- International Human Genome Sequencing Consortium Initial sequencing and analysis of the human genome. *Nature,* vol. 409, 860-921
- **KENAKIN T.** The measurement of efficacy in the drug discovery agonist selection process. *J. Pharmacol. Toxicol. Meth.,* 2000, vol. 42, 177-187
- **STABLES J ; GREEN A ; MARSHALL F ; FRASER N ; KNIGHT E ; SAUTEL M ; MILLIGAN G ; LEE M ; REES S.** A bioluminescent assay for agonist activity at potentially any G-protein-coupled receptor. *Anal Biochem,* 01 October 1997, vol. 252 (1), 115-26
- **SULLIVAN E ; TUCKER EM ; DALE IL.** Measurement of [Ca2+] using the Fluorometric Imaging Plate Reader (FLIPR. *Methods Mol Biol,* 1999, vol. 114, 125-33
- **PORTER RH ; BENWELL KR ; LAMB H ; MALCOLM CS ; ALLEN NH ; REVELL DF ; ADAMS DR ; SHEARDOWN MJ.** Functional characterization of agonists at recombinant human 5-HT2A, 5-HT2B and 5-HT2C receptors in CHO-K1 cells. *Br J Pharmacol,* September 1999, vol. 128 (1), 13-20
- **SALOMON Y.** Cellular responsiveness to hormones and neurotransmitters: conversion of [3H]adenine to [3H]cAMP in cell monolayers, cell suspensions, and tissue slices. *Methods Enzymol.,* 1991, vol. 195, 22-28
- **CHEN W. ; SHIELDS T.S. ; STORK P.J.S. ; CONE R.D.** A colorimetric assay for measuring activation of Gs- and Gq-coupled signaling pathways. *Anal Biochem.,* 1995, vol. 226, 349-54
- **POTENZA M.N. ; GRAMINSKI G.F. ; LERNER M.R.** A method for evaluating the effects of ligands upon Gs protein-coupled receptors using a recombinant melanophore-based bioassay. *Anal Biochem.,* 1992, vol. 206, 315-22
- **BRAUNER-OSBORNE H. ; BRANN M.R.** Pharmacology of muscarinic acetylcholine receptor subtypes (m1-m5): high throughput assays in mammalian cells. *Eur J Pharmacol.,* 1996, vol. 295, 93-102
- **BURSTEIN ES ; BRAUNER-OSBORNE H ; SPALDING TA ; CONKLIN BR ; BRANN MR.** Interactions of muscarinic receptors with the heterotrimeric G proteins Gq and G12: transduction of proliferative signals. *J Neurochem.,* 1997, vol. 68, 525-33
- **CONKLIN BR ; FARFEL Z ; LUSTIG KD ; JULIUS D ; BOURNE HR.** Substitution of three amino acids switches receptor specificity of Gq alpha to that of Gi alpha. *Nature,* 20 May 1993, vol. 363 (6426), 274-6
- **CONKLIN BR ; HERZMARK P ; ISHIDA S ; VOYNO-YASENETSKAYA TA ; SUN Y ; FARFEL Z ; BOURNE HR.** Carboxyl-terminal mutations of Gq alpha and Gs alpha that alter the fidelity of receptor activation. *Mol Pharmacol.,* 1996, vol. 50, 885-90
- **AMATRUDA TT 3RD ; STEELE DA ; SLEPAK VZ ; SIMON MI.** G alpha 16, a G protein alpha subunit specifically expressed in hematopoietic cells. *Proc Natl Acad Sci U S A,* 01 July 1991, vol. 88 (13), 5587-91
- **OFFERMANNS S ; SIMON MI.** G alpha 15 and G alpha 16 couple a wide variety of receptors to phospholipase C. *J Biol Chem,* 23 June 1995, vol. 270 (25), 15175-80
- **SIMON MI ; STRATHMANN MP ; GAUTAM N.** Diversity of G proteins in signal transduction. *Science,* 10 May 1991, vol. 252 (5007), 802-8
- **STRATHMANN MP ; SIMON MI.** G alpha 12 and G alpha 13 subunits define a fourth class of G protein alpha subunits. *Proc Natl Acad Sci U S A,* 01 July 1991, vol. 88 (13), 5582-6
- **GOMEZA J ; MARY S ; BRABET I ; PARMENTIER M ; RESTITUITO S ; BOCKAERT J ; PIN JP.** Coupling of metabotropic glutamate receptors 2 and 4 to G alpha 15, G alpha 16, and chimeric G alpha q/i proteins: characterization of new antagonists. *Mol Pharmacol,* October 1996, vol. 50 (4), 923-30
- **LEE JW ; JOSHI S ; CHAN JS ; WONG YH.** Differential coupling of mu-, delta-, and kappa-opioid receptors to G alpha16-mediated stimulation of phospholipase C. *J Neurochem.,* 1998, vol. 70, 2203-11

- **KOSTENIS E.** Is Galpha16 the optimal tool for fishing ligands of orphan G-protein-coupled receptors?. *Trends Pharmacol Sci,* November 2001, vol. 22 (11), 560-4
- **BOKOCH GM.** Interplay between Ras-related and heterotrimeric GTP binding proteins: lifestyles of the BIG and little. *FASEB J,* September 1996, vol. 10 (11), 1290-5
- **QUILLIAM LA ; REBHUN JF ; CASTRO AF.** A growing family of guanine nucleotide exchange factors is responsible for activation of Ras-family GTPases. *Prog Nucleic Acid Res Mol Biol.,* 2002, vol. 71, 391-444
- **CHERFILS J ; CHARDIN P.** GEFs: structural basis for their activation of small GTP-binding proteins. *Trends Biochem Sci.,* August 1999, vol. 24 (8), 306-11
- **SEIDEL MG ; KLINGER M ; FREISSMUTH M ; HOLLER C.** Activation of mitogen-activated protein kinase by the A(2A)-adenosine receptor via a rap1-dependent and via a p21(ras)-dependent pathway. *J Biol Chem,* 03 September 1999, vol. 274 (36), 25833-41
- **SCHMITT, J. M. ; P. J. S. STORK.** Beta-adrenergic receptor activates extracellular regulated kinases (ERKs) via the small G protein Rap1 and the serine/threonine kinase B-Raf. *J. Biol. Chem.,* 2000, vol. 275, 25342-25350
- **LOVA P ; PAGANINI S ; SINIGAGLIA ; BALDUINI C ; TORTI M.** A Gi-dependent pathway is required for activation of the small GTPase RaplB in human platelets. *J Biol Chem.,* 05 April 2002, vol. 277 (14), 12009-15
- **WOULFE D ; JIANG H ; MORTENSEN R ; YANG J ; BRASS LF.** Activation of RaplB by G(i) family members in platelets. *J Biol Chem.,* 2002, vol. 277 (26), 23382-90
- **GUO FF ; KUMAHARA E ; SAFFEN D.** A CalD-AG-GEFI/Rap1/B-Raf cassette couples M(1) muscarinic acetylcholine receptors to the activation of ERK1/2. *J Biol Chem,* 2001, vol. 276, 25568-25581
- **DE ROOIJ J ; ZWARTKRUIS FJT ; VERHEIJEN MHG ; COOL RH ; NIJMAN SMB ; WITTINGHOFER A ; BOS JL.** Epac is a Rap1 guanine-nueclotide-exchange factor directly activated by cyclic AMP. *Nature,* 1998, vol. 396, 474-477
- **KAWASAKI H ; SPRINGETT GM ; MOCHIZUKI N ; TOKI S ; NAKAYA M ; MATSUDA M ; HOUSMAN DE ; GRAYBIEL AM.** A family of cAMP-binding proteins that directly activate Rap1. *Science,* 1998, vol. 282, 2275-2279
- **MESSIER TL ; DORMAN CM ; BRAUNER-OSBORNE H ; EUBANKS D ; BRANN MR.** High throughput assays of cloned adrenergic, muscarinic, neurokinin, and neurotrophin receptors in living mammalian cells. *Pharmacol Toxicol,* 1995, vol. 76, 308-11
- **BURSTEIN E.S. ; SPALDING T.A. ; HILL-EUBANKS D. ; BRANN M.R.** Structure-function of muscarinic receptor coupling to G proteins. Random saturation mutagenesis identifies a critical determinant of receptor affinity for G proteins. *J Biol Chem.,* 1995, vol. 270, 3141-6
- **BURSTEIN ES ; HESTERBERG DJ ; GUTKIND JS ; BRANN MR ; CURRIER EA ; MESSIER TL.** The ras-related GTPase rac1 regulates a proliferative pathway selectively utilized by G-protein coupled receptors. *Oncogene,* 1998, vol. 24, 1617-23
- **ZHANG K ; NODA M ; VASS WC ; PAPAGEORGE AG ; LOWY DR.** Identification of small clusters of divergent amino acids that mediate the opposing effects of ras and Krev-1. *Science,* 1990, vol. 249, 162-5
- **SMIT MJ ; VERZIJL D ; IYENGAR R.** Identity of adenylyl cyclase isoform determines the rate of cell cycle progression in NIH 3T3 cells. *Proc Natl Acad Sci U S A,* 1998, vol. 95, 15084-9
- **LIM K. ; CHAE C.-B.** A Simple Assay for DNATransfection by Incubation of the Cells in Culture Dishes with Substrates for ☐-galactosidase. *Bio-Techniques,* 1989, vol. 7, 576-579
- **ALTSCHULER D ; LAPETINA EG.** Mutational analysis of the cAMP-dependent protein kinase-mediated phosphorylation site of Raplb. *J Biol Chem.,* 1993, vol. 268 (10), 7527-31
- **CHARDIN P ; CAMONIS JH ; GALE NW ; VAN AELST L ; SCHLESSINGER J ; WIGLER MH ; BAR-SAGI D.** Human Sos1: a guanine nucleotide exchange factor for Ras that binds to GRB2. *Science,* 28 May 1993, vol. 260 (5112), 1338-43
- **VAN DEN BERGHE N ; COOL RH ; WITTINGHOFER A.** Discriminatory residues in Ras and Rap for guanine nucleotide exchange factor recognition. *J Biol Chem.,* 16 April 1999, vol. 274 (16), 11078-85
- **LUSTIG KD ; CONKLIN BR ; HERZMARK P ; TAUSSIG R ; BOURNE HR.** Type II adenylylcyclase integrates coincident signals from Gs, Gi, and Gq. *J Biol Chem,* 05 July 1993, vol. 268 (19), 13900-5
- **BURSTEIN ES ; SPALDING TA ; BRANN MR.** Pharmacology of muscarinic receptor subtypes constitutively activated by G proteins. *Mol Pharmacol.,* February 1997, vol. 51 (2), 312-9
- **BURSTEIN ES ; SPALDING TA ; BRAUNER-OSBORNE H ; BRANN MR.** Constitutive activation of muscarinic receptors by the G-protein Gq. *FEBS Lett.,* 24 April 1995, vol. 363 (3), 261-3
- **SONG ZH ; MODI W ; BONNER TI.** Molecular cloning and chromosomal localization of human genes encoding three closely related G protein-coupled receptors. *Genomics,* 20 July 1995, vol. 28 (2), 347-9

- **EGGERICKX D ; DENEF JF ; LABBE O ; HAYASHI Y ; REFETOFF S ; VASSART G ; PARMENTIER M ; LIBERT F.** Molecular cloning of an orphan G-protein-coupled receptor that constitutively activates adenylate cyclase. *Biochem J,* 01 August 1995, vol. 309 (3), 837-43
- **UHLENBROCK K ; GASSENHUBER H ; KOSTENIS E.** Sphingosine 1-phosphate is a ligand of the human gpr3, gpr6 and gpr12 family of constitutively active G protein-coupled receptors. *Cell Signal.,* November 2002, vol. 14 (11), 941
- **DONG X ; HAN S ; ZYLKA MJ ; SIMON MI ; ANDERSON DJ.** A diverse family of GPCRs expressed in specific subsets of nociceptive sensory neurons. *Cell,* 07 September 2001, vol. 106 (5), 619-32
- **CHAN AM ; FLEMING TP ; MCGOVERN ES ; CHEDID M ; MIKI T ; AARONSON SA.** Expression cDNA cloning of a transforming gene encoding the wild-type G alpha 12 gene product. *Mol Cell Biol,* February 1993, vol. 13 (2), 762-8
- **XU N ; VOYNO-YASENETSKAYA T ; GUTKIND JS.** Potent transforming activity of the G13 alpha subunit defines a novel family of oncogenes. *Biochem Biophys Res Commun,* 15 June 1994, vol. 201 (2), 603-9
- **WU J ; DENT P ; JELINEK T ; WOLFMAN A ; WEBER MJ ; STURGILL TW.** Inhibition of the EGF-activated MAP kinase signaling pathway by adenosine 3',5'-monophosphate. *Science,* 1993, vol. 262, 1065-9
- **COOK SJ ; MCCORMICK F.** Inhibition by cAMP of Ras-dependent activation of Raf. *Science,* 1993, vol. 262, 1069-72
- **SCHMITT JM ; STORK PJ.** Cyclic AMP-mediated inhibition of cell growth requires the small G protein Rap1. *Mol Cell Biol.,* 2001, vol. 11, 3671-83
- **BOS JL ; DE ROOIJ J.** Reedquist KA Rap1 signaling: adhering to new models. *Nat Rev Mol Cell Biol.,* 2001, vol. 2, 369-77
- **KITAYAMA H ; SUGIMOTO Y ; MATSUZAKI T ; IKAWA Y ; NODA M.** A ras-related gene with transformation suppressor activity. *Cell,* 1989, vol. 56, 77-84
- **YORK RD ; YAO H ; DILLON T ; ELLIG CL ; ECKERT SP ; MCCLESKEY EW ; STORK PJS.** Rap1 mediates sustained MAP kinase activation induced by nerve growth factor. *Nature,* 1998, vol. 392, 622-626
- **WU C ; LAI CF ; MOBLEY WC.** Nerve growth factor activates persistent Rap1 signaling in endosomes. *J Neurosci.,* 01 August 2001, vol. 21 (15), 5406-16
- **M'RABET L ; COFFER P ; ZWARTKRUIS F ; FRANKE B ; SEGAL AW ; KOENDERMAN L ; BOS JL.** Activation of the small GTPase rap1 in human neutrophils. *Blood,* 15 September 1998, vol. 92 (6), 2133-40
- **MCLEOD SJ ; INGHAM RJ ; BOS JL ; KUROSAKI T ; GOLD MR.** Activation of the Rap1 GTPase by the B cell antigen receptor. *J Biol Chem.,* 30 October 1998, vol. 273 (44), 29218-23
- **KATAGIRI K ; HATTORI M ; MINATO N ; KINASHI T.** Rap1 functions as a key regulator of T-cell and antigen-presenting cell interactions and modulates T-cell responses. *Mol Cell Biol.,* February 2002, vol. 22 (4), 1001-15
- **REEDQUIST, K. A. ; E. ROSS ; E. A. KOOP ; R. M. WOLTHUIS ; F. J. ZWARTKRUIS ; Y. VAN KOOYK ; M. SALMON ; C. D. BUCKLEY ; J. L. BOS.** The Small GTPase, Rap1, mediates CD31-induced integrin adhesion. *J. Cell Biol.,* 2000, vol. 148, 1151-1158
- **OSTROM RS ; VIOLIN JD ; COLEMAN S.** Insel PA Selective enhancement of beta-adrenergic receptor signaling by overexpression of adenylyl cyclase type 6: colocalization of receptor and adenylyl cyclase in caveolae of cardiac myocytes. *Mol Pharmacol.,* 2000, vol. 57, 1075-9
- **KOCH WJ ; HAWES BE ; ALLEN LF ; LEFKOWITZ RJ.** Direct evidence that Gi-coupled receptor stimulation of mitogen-activated protein kinase is mediated by G beta gamma activation of p21ras. *Proc Natl Acad Sci U S A.,* 20 December 1994, vol. 91 (26), 12706-10